# EUROPÄISCHE PATENTANMELDUNG

(11) **EP 2 546 265 A1**
(43) Veröffentlichungstag der Anmeldung: **16.01.2013**
(21) Anmeldenummer: 12075047.6
(22) Anmeldetag: 16.05.2012
(51) Int. Cl.: C07K 16/00, C12P 21/00

(54) **Speziell für Disulfidbrücken-enthaltende Proteine geeignete Methode zur eukaryontischen zellfreien Proteinsynthese**

(30) Priorität: 11.07.2011 DE 102011107562
(71) Anmelder: RiNA Netzwerk Technologien GmbH, 14195 Berlin (DE)
(72) Erfinder: Merk, Helmut, 10551 Berlin (DE); Stiege, Wolfgang, Dr., 12163 Berlin (DE); Gless, Christine, 10997 Berlin (DE); Gerrits, Michael, Dr., 12351 Berlin (DE)
(74) Vertreter: Jungblut, Bernhard Jakob

(57) **Zusammenfassung**

Die Erfindung betrifft ein Verfahren zur Herstellung von monomeren oder dimeren Proteinen oder Peptiden, enthaltend interne oder externe Disulfidbrücken mit den folgenden Verfahrensstufen: a) es wird eine zellfreies Lysat, erhältlich aus eukaryotischen Zellen, bereit gestellt, welches funktionale mikrosomale Vesikel enthält, b) zum Lysat wird eine Nukleinsäure zugegeben, welche das Protein oder Peptid codiert und zusätzlich eine Signalsequenz enthält, c) das Lysat mit der Nukleinsäure wird für eine vorgegebene Zeit auf einer Temperatur im Bereich von 20-35°C gehalten, wobei mit der Nukleinsäure gebildete Proteine oder Peptide in die mikrosomalen Vesikel translociert werden, d) die mikrosomalen Vesikel werden anschließend aufgelöst und die dabei erhaltenen Proteine oder Peptide werden optional von dem Lysat abgetrennt.

## Beschreibung

### Gebiet der Erfindung

Die Erfindung betrifft ein Verfahren zur Herstellung von monomeren oder dimeren Proteinen oder Peptiden, enthaltend interne oder externe Disulfidbrücken mit den folgenden Verfahrensstufen: a) es wird eine zellfreies Lysat bereit gestellt, zum Lysat wird eine Nukleinsäure zugegeben, welche das Protein oder Peptid codiert, c) das Lysat mit der Nukleinsäure wird für eine vorgegebene Zeit auf einer Temperatur im Bereich von 20-35°C gehalten, d) die dabei erhaltenen Proteine oder Peptide werden optional von dem Lysat abgetrennt. Die Erfindung betrifft desweiteren Proteine oder Peptide erhältlich mit einem solchen Verfahren sowie eine Zubereitung erhältlich mit einem solchen Verfahren.

### Stand der Technik und Hintergrund der Erfindung

Monoklonale Antikörper werden in einem breiten Gebiet von Applikationen eingesetzt, welche auch die Verwendung als Forschungswerkzeuge sowie diagnostische und therapeutische Zwecke einschließt.

Da scFv (single-chain variable fragment, Einzelstrangantikörper) und Fab (antigen-binding fragment, antigenbindendes Fragment) als Teile von kompletten Antikörpern klein und in Escherichia coli exprimierbar sind und ein Durchmustern sowie eine Selektion auf hochaffin bindende Moleküle erlauben, weitet sich der mögliche Bereich für Applikationen dieser Moleküle schnell aus. Eine der wichtigsten Limitierungen dieser Ausweitung ist jedoch die Geschwindigkeit der Generierung und die Analyse der Effizienz der Bindung und Wirkung von Antikörpern auf ihre Zielmoleküle. Eine Beschleunigung der Antikörperproduktion würde letztlich ein schnelleres Reagieren auf gesundheitliche Bedrohungen, verursacht durch pandemische Verbreitung von pathogenen Viren und Mikroorganismen ermöglichen.

Die konventionelle Herstellung von Antikörpern erfolgt über die Kultivierung tierischer Zellen (Nilsang S, Nandakumar KS, Galaev IY, Rakshit SK, Holmdahl R, Mattiasson B, Kumar A. (2007) Monoclonal antibody production using a new supermacroporous cryogel bioreactor. Biotechnol Prog 23:932-939; Rodrigues ME, Costa AR, Henriques M, Azeredo J, Oliveira R (2010) Technological progresses in monoclonal antibody production systems. Biotechnol Prog 2:332-51). Eine deutliche Beschleunigung sowie eine ökonomischere Produktion von Antikörpern konnte in den letzten Jahren durch deren Expression in Escherichia coli Zellen erzielt werden (Humphreys DP (2003) Production of antibodies and antibody fragments in Escherichia coli and a comparison of their functions, uses and modification. Curr Opin Drug Discov Devel 6:188-196). Zellbasierte Methoden der Antikörperherstellung erfordern jedoch eine zeit- und arbeitsintensive Zellkulturführung. Für eine bakterielle Expression sind darüber hinaus weitere Arbeiten für die Klonierung der für Antikörper kodierenden, exprimierbaren Matrizen notwendig (Hoogenboom HR (2005) Selecting and screening recombinant antibody libraries. Nat Biotechnol 23:1105-1116).

Die zellfreie Proteinexpression wird dagegen inzwischen als vielversprechende Alternative zur Überwindung der Limitierungen zellbasierten Methoden angesehen. Ein Grund hierfür liegt in der in den letzten Jahren erzielten, deutlichen Verbesserung der Produktivität, Ökonomie sowie der Aktivität der synthetisierten Protein in zellfreien prokaryotischen (Spirin AS, Baranov VI, Ryabova LA, Ovodov SY, Alakhov YB (1988) A continuous cell-free translation system capable of producing polypeptides in high yield. Science 242:1162-4; Strey J, Merk H, Stiege W (2004) Verfahren zur präparativen in vitro Proteinbiosynthese. Patent DE 102004032460; Kim TW, Oh IS, Keum JW, Kwon YC, Byun JY, Lee KH, Choi CY, Kim DM (2007) Prolonged cell-free protein synthesis using dual energy sources: combined use of creatine phosphate and glucose for the efficient supply of ATP and retarded accumulation of phosphate. Biotechnol Bioeng 97:1510-1515) und eukaryotischen Systemen (Madin K, Sawasaki T, Ogasawara T, Endo Y (2000) A highly efficient and robust cell-free protein synthesis system prepared from wheat embryos: plants apparently contain a suicide system directed at ribosomes. Proc Natl Acad Sci USA 97(2): 559-564; Tsuboi T, Takeo S, Iriko H, Jin L, Tsuchimochi M, Matsuda S, Han E, Otsuk, H, Kaneko O, Sattabongkot J, Udomsangpetch R, Sawasaki T, Tori, M, Endo, Y (2008) Wheat germ Cell-Free System-Based Production of Malaria Proteins for Discovery of Novel Vaccine Candidates. Infection and Immunity 1702-1708; Tarui H, Murata M, Tani I, Imanishi S, Nishikawa S, Hara T (2001) Establishment and characterization of cell-free translation/glycosylation in insect cell (Spodoptera frugiperda 21) extract prepared with high pressure treatment. Appl Microbiol Biotechnol 55:446-453; Kubick S, Schacherl J, Fleischer-Notter H, Royall E, Roberts LO, Stiege W (2003) In vitro Translation in an Insect-Based Cell-Free System. In: Swartz, J.R. (Ed.) Cell-Free Protein Expression. Springer, Berlin Heidelberg New York, 209-217; Mikami S, Masutani M, Sonenberg N, Yokoyama S, Imataka H (2006) An efficient mammalian cell-free translation system supplemented with translation factors. Protein Expr Purif 46(2): 348-357). Modifizierungen einiger dieser Systeme ermöglichten die Darstellung disulfidverbrückter Proteine inklusive scFv (Einzelstrangantikörper) (Ryabova LA, Desplancq D, Spirin AS, Pluckthun A (1997) Functional antibody production using cell-free translation: effects of protein disulfide isomerase and chaperones. Nat Biotechnol 15:79-84; Merk H, Stiege W, Tsumoto K, Kumagai I, Erdmann VA (1999) Cell-free Expression of two Single-Chain Monoclonal Antibodies against Lysozyme: Effect of Domain Arrangement on the Expression. J Biochem 125:328-333). Fab Antikörperfragmente sind jedoch aufgrund ihrer besseren Bindungseigenschaften attraktiver als scFv. Kürzlich wurde die zellfreie Expression eines Fab in einem auf E. coli basierenden System gezeigt (Oh IS, Lee JC, Lee MS, Chung JH, Kim DM (2010) Cell-free production of functional antibody fragments. Bioprocess Biosyst Eng 33:127-32)

Nachteile der auf E. coli basierenden zellfreien Systeme sind allerdings eine geringe spezifische Aktivität des hergestellten Fab sowie die Erfordernis der Entfernung von Toxinen des Expressionssystems, bevor das synthetisierte Fab für einen zellbasierten Qualitätstest eingesetzt werden kann. E. coli Systeme liefern in ihrer einfachen Ausführung (batch System) inzwischen Syntheseleistungen von bis zu 1 Milligramm neu synthetisertem Protein je Milliliter Reaktionslösung. Der höchste Wert für die Ausbeute an funktionellem Fab aus einem zellfreien System liegt jedoch nur bei 30 *µ*g/ml (Oh IS, Lee JC, Lee MS, Chung JH, Kim DM (2010) Cell-free production of functional antibody fragments. Bioprocess Biosyst Eng 33:127-32) Trotz der hohen Produktivität des zellfreien E. coli Systems ist damit nur ein Bruchteil des synthetisierten Proteins verwendbar. Darüber hinaus muss, um genaue Messergebnisse erhalten zu können, der aktive Anteil des synthetisierten Proteins vom inaktiven Anteil abgetrennt werden, was einen zusätzlichen Arbeitsaufwand für diesen Reinigungsschritt bedeutet.

Auf E. coli basierende zellfreie Systeme weisen hohe Akivitäten auf, welche im Verlauf der Reaktion das Redoxpontenzial stark verändern. Diese Veränderung verringert die Aktivität der synthetisiertern Antikörperfragmente. Eine Verringerung der Veränderung des Redoxpotenzials wurde durch chemische Vorbehandlung des Zelllysats erzielt Kim DM, Swartz JR (2004) Efficient production of a bioactive, multiple disulfide-bonded protein using modified extracts of Escherichia coli. Biotechnol Bioeng 85:122-9; Oh IS, Kim DM, Kim TW, Park CG, Chloi CY (2006) Providing an oxidizing environment for the cell-free expression of disulfide-containing proteins by exhausting the reducing activity of Escherichia coli S30 extract. Biotechnol Prog 22:1225-89. Dies wirkt sich jedoch nachteilig auf die Handhabung des Systems aus, da ein weiterer Arbeitsschritt erforderlich ist, welcher sich darüber hinaus nur mit Zusatzaufwand automatisieren lässt.

Um in E. coli basierten zellfreien Systemen nennenswerte Konzentrationen an löslichem und aktivem Antikörperfragment synthetisieren zu können, werden diese mit Chaperonen, beispielsweise GroE, DnaK und Proteindisulfidisomerase, beispielsweise PDI, DsbC supplementiert (Ryabova LA, Desplancq D, Spirin AS, Pluckthun A (1997) Functional antibody production using cell-free translation: effects of protein disulfide isomerase and chaperones. Nat Biotechnol 15:79-84; Merk H, Stiege W, Tsumoto K, Kumagai I, Erdmann VA (1999) Cell-free Expression of two Single-Chain Monoclonal Antibodies against Lysozyme: Effect of Domain Arrangement on the Expression. J Biochem 125:328-333; Tsumoto K, Nakaoki Y, Ueda Y, Ogasahara K, Yutani K, Watanabe K, Kumagai I (1994) Effect of the order of antibody variable regions on the expression of the single-chain HyHEL10 Fv fragment in E. coli and the thermodynamic analysis of its antigen-binding properties. Biochem Biophys Res Commun 201:546-51; Oh IS, Lee JC, Lee MS, Chung JH, Kim DM (2010) Cell-free production of functional antibody fragments. Bioprocess Biosyst Eng 33:127-32; Kim DM, Swartz JR (2004) Efficient production of a bioactive, multiple disulfide-bonded protein using modified extracts of Escherichia coli. Biotechnol Bioeng 85:122-9; Oh IS, Kim DM, Kim TW, Park CG, Chloi CY (2006) Providing an oxidizing environment for the cell-free expression of disulfide-containing proteins by exhausting the reducing activity of Escherichia coli S30 extract. Biotechnol Prog 22:1225-8).

### Technisches Problem der Erfindung

Der Erfindung liegt daher das technische Problem zu Grunde, ein Verfahren zur Herstellung von Proteinen und Peptiden, insbesondere Antikörpern oder Antikörperfragmenten anzugeben, wobei die erhaltene Proteine oder Peptide eine verbessert hohe spezifische Aktivität ausweisen und zugleich der Syntheseaufwand, insbesondere auch bezüglich der eingesetzten Nukleinsäure sowie der Nachbehandlung der erhaltenen Proteine oder Peptide, reduziert ist.

### Grundzüge der Erfindung und bevorzugte Ausführungsformen

Zur Lösung dieses technischen Problems lehrt die Erfindung ein Verfahren zur Herstellung von monomeren oder dimeren Proteinen oder Peptiden, enthaltend interne oder externe Disulfidbrücken mit den folgenden Verfahrensstufen: a) es wird eine zellfreies Lysat, erhältlich aus eukaryotischen Zellen, bereit gestellt, welches funktionale mikrosomale Vesikel enthält, b) zum Lysat wird eine Nukleinsäure zugegeben, welche das Protein oder Peptid codiert und zusätzlich eine Signalsequenz enthält, c) das Lysat mit der Nukleinsäure wird für eine vorgegebene Zeit auf einer Temperatur im Bereich von 20-35°C gehalten, wobei mit der Nukleinsäure gebildete Proteine oder Peptide in die mikrosomalen Vesikel translociert werden, d) die mikrosomalen Vesikel werden anschließend aufgelöst und die dabei erhaltenen Proteine oder Peptide werden optional von dem Lysat abgetrennt.

Die erfindungsgemäße Verfahren unterscheidet sich vom Stand der Technik in mehreren Aspekten. Anstelle der bislang für die zellfreie Synthese von Fab Antikörperfragmenten verwendeten prokaryotischen E. coli Zellen werden hier eukaryotische Zellen für die Herstellung des Expressionssystems eingesetzt. Bislang findet die zellfreie Synthese von Fab in einem Milieu statt, welches dem Zytosol einer lebenden Zelle entspricht. Bei der hier beschriebenen Erfindung enthalten die für Antikörperfragmente kodierenden Matrizen Signalpeptidsequenzen. Mit Hilfe dieser Signalpeptidsequenzen werden die Antikörperfragmente kotranslational über natürliche Mechanismen in mikrosomale Vesikel transloziert, welche dem Endoplasmatischen Retikulum entstammen. Nach dem Stand der Technik für die zellfreie Proteinsynthese in einem System aus Insektenzellen (TNT^{®} T7 Insect Cell Extract Protein, Promega # L1101, L1102 und EasyXpress Insect Kit II, Qiagen # 32561, 32562) findet die Proteinsynthese bei 28-30°C für eine bis vier Stunden statt. In dem hier beschriebenen System findet die Synthese bei tieferer Inkubationstemperatur von beispielsweise 25°C und einer Inkubationszeit von vier bis fünf Stunden oder weniger statt. Im Anschluss an die Synthesereaktion werden die mikrosomalen Vesikel, in denen sich synthetisiertes Fab befindet, mit Hilfe von Detergens aufgelöst und auf diese Weise Fab freigesetzt. Bislang werden vorgefertigte mRNA oder zirkuläre DNA kodierend für Fab als Matrizen für die Proteinsynthese eingesetzt. In dem hier beschriebenen Verfahren kann auch lineare, über PCR (Polymerasekettenreaktion) generierte DNA direkt, ohne vorherige Aufreinigung als Matrize verwendet werden. Dabei kann die hier eingesetzte PCR-generierte Matrize sowohl in einem prokaryotischen, als auch in eukaryotischen Systemen basierend auf Insektenzellen (beispielsweise Spodoptera frugiperda) und auf Säugetierzellen (Chinese Hamster Ovary) als Matrize verwendet werden. Bei dem hier beschriebenen System wird das für die Proteinsynthese verwendete Lysat aus Insektenzellen im Gegensatz zum Stand der Technik in einem nicht-reduzierenden Milieu hergestellt. Die Synthese von Fab in diesem Insektenzelllysat findet abweichend vom Stand der Technik nicht in reduzierendem sondern in einem definierten Redoxmilieu statt. Abweichend zum Stand der Technik wird das Zelllysat vorzugsweise nicht chemisch vorbehandelt, um Aktivitäten zu unterdrücken, welche das Redoxpotenzial im Verlauf der Reaktion stark verändern. Im Gegensatz zum Stand der Technik wird das hier beschriebene System vortzugsweise weder mit Chaperonen noch mit Proteindisulfidisomerase supplementiert.

Basierend auf diesen Unterschieden werden verschiedene Vorteile erzielt. Die spezifische Aktivität der gemäß dieser Erfindung hergestellten Proteine bzw. Antikörper oder Antikörperfragmente ist gegenüber dem Stand der Technik wesentlich höher. Während ein prokaryotisches zellfreies System im Vergleich zu einer typischen Proteinsyntheseleistung von mehreren hundert Mikrogramm bis zu einem Milligramm je Milliliter Reaktionslösung nur etwa 30 Mikrogramm je Milliliter Reaktionslösung und damit einen sehr geringen Prozentsatz an aktivem Fab liefert, werden Proteine bzw. Antikörper oder Antikörperfragmente mit der hier beschriebenen Erfindung überwiegend in aktiver Form produziert. Dies hat beim Stand der Technik zur Folge, dass für eine Anwendung, bei der der inaktive Anteil der Antikörperfragmente störend wirkt, dieser Anteil vor der Anwendung durch zusätzliche Arbeitsschritte abgetrennt werden muss. Solche zusätzlichen Arbeitsschritte erschweren eine Automatisierung einer Antikörperfragmentherstellung deutlich. Eine störende Wirkung von inaktivem Antikörperfragment kann beispielsweise die Quantifizierung von synthetisiertem, aktivem Antikörperfragment über Immunodetektion (Western Blot oder Enzyme Linked Immuno Sorbant Assay) sein, da diese Methode normalerweise keine Unterscheidung von aktivem und inaktivem Protein zulässt. Gegenüber dem Stand der Technik bezüglich des hier verwendeten zellfreien Proteinsynthesesystems basierend auf Insektenzellen ist der Vorteil des hier beschriebenen Systems, dass nur in diesem aktive Fab Antikörperfragmente hergestellt werden können. Das hier beschriebene Verfahren erlaubt die Synthese von Antikörperfragment in einem vollständig zellfreien Milieu, da auch die Generierung der für Antikörperfragment kodierenden Matrize im Gegensatz zum Stand der Technik über die zellfreie Methode der PCR erfolgen kann. Dies hat mehrere Vorteile. Der Zeit- und Arbeitsaufwand für die Generierung der Matrizen und der Antikörperfragmente verringert sich von etwa ein bis zwei Wochen auf ein bis zwei Tage. Da bei der hier beschriebenen Herstellung von Antikörperfragmenten in keinem Arbeitsschritt lebende Zellen erforderlich sind und keine rekombinante Plasmid-DNA hergestellt werden muss, entfallen Risiken und gesetzliche Beschränkungen im Umgang mit gentechnisch veränderten Organismen. Gemäß dem Stand der Technik werden Antikörperfragmente zellfrei bislang in Systemen basierend auf E. coli Zellen hergestellt. Solche Systeme enthalten Endotoxine. In solchen Systemen hergestellte Antikörperfragmente können nicht direkt für Assays basierend auf eukaryotischen beispielsweise humanen Zellen verwendet werden, da Endotoxine normalerweise zytotoxisch auf diese Zellen wirken und die Messung des spezifischen Effekts von Antikörperfragment auf diese Zellen durch den zytotoxischen Effekt überdeckt wird. Um Antikörperfragmente aus E. coli basierten Systemen in solchen Assays sinnvoll einsetzen zu können, müssen die Antikörperfragmente zuvor durch zusätzliche Arbeitsschritte gereinigt werden. Dies wirkt sich nachteilig auf die Arbeitszeit sowie auf eine Automatisierbarkeit der Herstellung von Antikörperfragmenten aus. Da das in dieser Erfindung verwendete System keine Endotoxine enthält, bestehen diese Probleme hier nicht. Das Zelllysat des hier beschriebenen Systems muss nicht chemisch vorbehandelt werden, um Aktivitäten zu unterdrücken, welche das Redoxpotenzial im Verlauf der Reaktion stark verändern würden. Es kann ohne den Zusatz reduzierender Chemikalien zum Lysat gearbeitet werden. Dies vereinfacht die Handhabung des Systems im Allgemeinen und speziell im Hinblick auf die Automatisierbarkeit der Proteinsynthesereaktion. Das hier beschriebene System erfordert keine Herstellung und Supplementierung von Chaperonen und Proteindisulfidisomerase. Es ist damit einfacher aufgebaut und bezüglich dieser Komponenten ökonomischer als Systeme, die dem Stand der Technik entsprechen.

Im Rahmen der Erfindung sind verschiedene Weiterbildungen möglich. Vorzugsweise sind die Proteine oder Peptide vorzugsweise dimere monoklonalen Antikörper oder physiologisch wirksame vorzugsweise dimere Antikörperfragmente. Aufgrund der Disulfidbrücken innerhalb solcher Strukturen wirken sich die Vorteile der Erfindung besonders stark aus.

Als eukaryotische Zellen kommen insbesondere Insektenzellen, aber auch HeLa-Zellen, CHO (Chinese Hamster Ovary) Zellen, HEK (Human Embryonic Kidney) Zellen, Weizenkeim-Zellen, Kaninchen Retikulozyten Zellen, Hefe-Zellen (z.B. Saccharomyces cerevisiae), Protozoen (z.B. Leishmania) Zellen oder Grünalgen in Frage. Unter den Insektenzellen können beispielsweise Zellen der Spezies Spodoptera frugiperda, Drosophila melanogaster oder Trichoplusia ni eingesetzt werden.

Als Signalpeptide sind im Falle der Insektenzellen neben Melittin aus Honigbienen PPD1, diphosphonucleotide phosphatase/phosphodiesterase aus Lupinus luteus, Azurocidin aus Homo sapiens oder gp67 aus Autographa californica einsetzbar. Für andere Zellspecies sind typische Signalpeptide: a) Proteinsynthesesysteme aus Säugetierzellen (z.B. HeLa, CHO, HEK, Kaninchen Retikulozyten) die Sequenzen aus: Luciferase aus Gaussia, Luciferase aus Metridia, Luciferase aus Vargula, Chymotrypsinogen aus Homo sapiens, Humanem Interleukin-2 aus Homo sapiens, Humanem Trypsinogen-2 aus Homo sapiens, Oikosin 1 aus Oikopleura dioica, b) für Proteinsynthesesysteme aus Hefen (z.B. Saccharomyces cerevisiae): Inulinase aus Kluyveromyces marxianus, Xylanase aus Aureobasidium pullulans, c) für Proteinsynthesesysteme aus Algen die Sequenzen aus: Calreticulin aus Bigelowiella natans, Protein disulfide isomerase Bigelowiella natans, d) für Proteinsynthesesysteme aus Protozoen (z.B. Leishmania) die Sequenzen aus: gp63 aus Leishmania, IFN-7 aus Mus musculus ergänzen. Die im Verfahren einzusetzende Nukleinsäure enthält dann neben der für das Protein bzw. Peptid codierenden Sequenz eine für das Signalpeptid codierende zusätzliche Sequenz. Letztere ist meist N-terminal der für das Protein/Peptid codierenden Sequenz angeordnet, kann jedoch grundsätzlich auch innerhalb dieser oder C-terminal liegen.

In der Stufe d) können alle fachüblichen Substanzen, insbesondere Detergentien eingesetzt werden. Neben Brij-35 sind hier beispielhaft zu nennen Triton X-100, NP40 (Nonidet P40), Tween 20, CHAPS (3-[(3-cholamidopropyl)dimethylammonio] -1-propanesulfonate), DDM (n-Dodecyl-beta-D-maltoside), Digitonin oder Glucopyranoside.

Ansonsten finden alle fachüblichen Methoden und Mittel, beispielsweise zur Abtrennung von Proteinen oder Peptiden von Lysaten, sofern gewünscht, Anwendung.

Die Erfindung betrifft desweiteren Proteine oder Peptide oder Zubereitung enthaltend Proteine oder Peptide, erhältlich mit einem erfindungsgemäßen Verfahren, sowie eine Zubereitung (beispielsweise erhalten in Stufe c) oder d)) enthaltend Proteine oder Peptide und ein zellfreies Lysat, erhältlich aus eukaryotischen Zellen, wobei das Lysat funktionale mikrosomale Vesikel oder Produkte einer Auflösung solcher mikrosomalen Vesikel enthält, und wobei die Zubereitung keine (endogenen und/oder zugesetzten) Chaperone und/oder Proteindisulfidisomerasen enthält. Die Ausführungen betreffend das Verfahren gelten analog.

Im Folgenden wird die Erfindung anhand von Ausführungsbeispielen näher erläutert.

### Beispiel 1

In den Ausführungsbeispielen wird Zelllysat aus Spodoptera frugiperda Insektenzellen wie in der Literaturstelle Kubick S, Schacherl J, Fleischer-Notter H, Royall E, Roberts LO, Stiege W (2003) In vitro Translation in an Insect-Based Cell-Free System. In: Swartz, J.R. (Ed.) Cell-Free Protein Expression. Springer, Berlin Heidelberg New York, 209-217, beschrieben hergestellt mit dem Unterschied, dass der für die Chromatographie des Zentrifugationsüberstands des Zelllysats verwendete Elutionspuffer kein Reduktionsmittel enthält.

Im Anschluss wird das Lysat mit einer Endkonzentration von 6,9 U S7 Micrococcal nuclease (Roche) je Milliliter Lysat bei einer Endkonzentration von 1 mM CaCl₂ für 20 Minuten bei 20°C inkubiert und der Verdau durch Einstellung einer Endkonzentration von 5 mM EGTA und Kühlung auf Eis gestoppt. Gekoppelte Transkriptions-/Translationsreaktionen enthalten 35 Vol% nukleasebehandeltes Zelllysat und des Weiteren 30 mM Hepes/KOH pH 7,6, 2,9 mM Magnesiumacetat, 75 mM Kaliumacetat, 0,25 mM Spermidin, 20 mg/ml Creatinphosphat, 1,75 mM ATP, 0,3 mM CTP, 0,3 mM GTP, 0,3 mM UTP, 0,33 mM P1,P3-Di-(Guanosin-5-)Triphosphat, 2,5 mM Glutathion in oxidierter Form, 0,5 mM Glutathion in reduzierter Form, 100 U/ml Ribonukleaseinhibitor RNasin (Promega), 50 U/*µ*l T7 RNA-Polymerase (Roche), 17,5 *µ*g/ml tRNA aus Bäckerhefe (Roche), 0,1 mM jeweils aller 20 natürlichen Aminosäuren, 0,1 mg/ml Creatinkinase (Roche) und jeweils 7,5 *µ*g/ml Plasmid DNA kodierend für leichte und schwere Kette eines Fab Antikörperfragments. Bei Bedarf wird der Reaktion L-[U-14C] Leucin (GE Healthcare) zur Einstellung einer molaren Aktivität von 40 dpm/pmol hinzugefügt. Die Reaktionsansätze werden für 4 Stunden bei 25°C unter Schütteln bei 700 rpm im Thermomixer (Eppendorf) inkubiert.

Im Anschluss an die Synthesereaktion wird dem Reaktionsansatz zur Lyse der Fab enthaltenden mikrosomalen Vesikel mit Brij-35 Lösung in einer Endkonzentration von 0,05% versetzt und 5 min bei Raumtemperatur inkubiert.

### Beispiel 2

Anti-Hühnereiweis-Lysozym Fab Antikörperfragment sowie Anti-humanes CD4 Fab Antikörperfragment werden wie in Ausführungsbeispiel 1 beschrieben synthetisiert. Als Matrizen werden die Plasmide pIX5.0-Mel-LaLys und pIX5.0-HaLys (anti-Lysozym) sowie die linearen, mit dem EasyXpress Linear Template Kit plus (Qiagen) generierten DNA Matrizen Mel-VLCL-SII und Mel-VHCH1 (anti-CD4), welche gemäß der hier beschriebenen Erfindung für ein Signalpeptid am N-Terminus der Fab Ketten kodieren, eingesetzt.

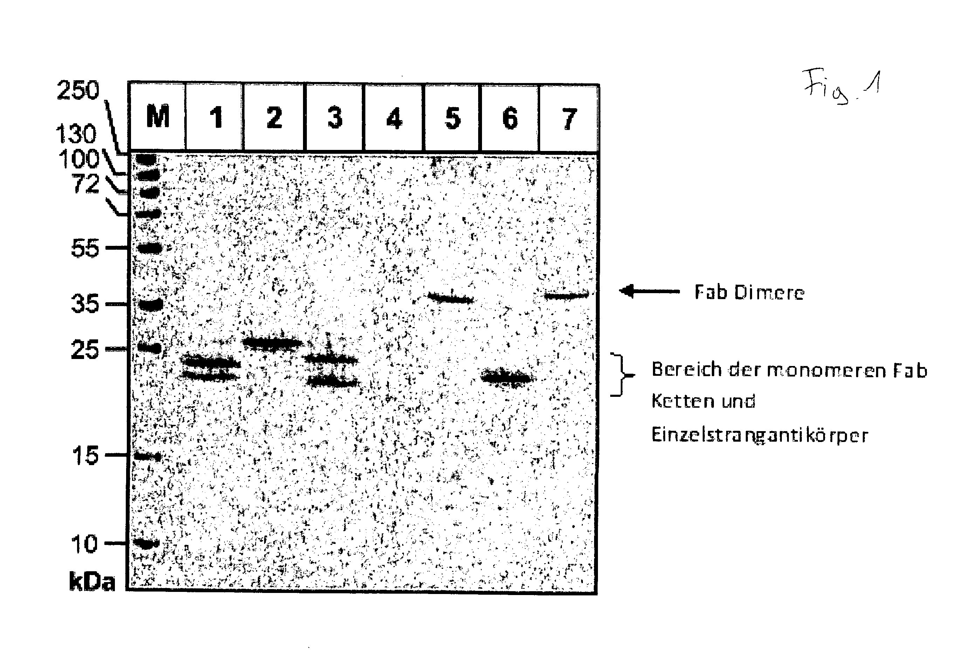

Nach der Reaktion wurden von jeder Synthesereaktion jeweils zwei 5 *µ*l Aliquots mit Aceton präzipitiert. Von den jeweils zwei resultierenden Pellets wurde eines in SDS-Probenpuffer mit Reduktionsmittel gelöst und Disulfidbrücken durch Erhitzen auf 90°C für 3 Minuten gespalten. Das jeweils zweite Pellet wurde in SDS-Probenpuffer ohne Reduktionsmittel durch 30 Minuten Inkubation bei 37°C gelöst, ohne entstandene Disulfidbrücken zu spalten. Abbildung 1 zeigt das Ergebnis der Analyse als Autoradiogramm nach SDS PAGE.

Demnach sind im Autoradiogramm bei den mit Reduktionsmittel behandelten Proben jeweils die koexprimierten leichten und schweren Ketten als Banden mit erwartetem Molekulargewicht zu sehen, während unter nicht-reduzierenden Bedingungen Banden mit Molekulargewicht detektiert werden, wie es für Fab Dimer erwartet wird. Die monomeren Ketten wurden dabei im Proteinsynthesesystem überwiegend zu Dimeren umgesetzt.

Abb. 1 zeigt im Einzelnen die Dimerisierung von Fab Ketten über Disulfidverbrückung. Anti-Hühnereiweis-Lysozym Fab Antikörperfragment und Anti-humanes CD4 Fab Antikörperfragment wurden zellfrei gemäß der hier beschriebenen Erfindung synthetisiert und die Verknüpfung der einzelnen leichten und schweren Immunglobulinketten zum disulfidverbrückten Dimer gelelektrophoretisch analysiert. Spuren 1-3: Die Disulfidbrücken der Fab Ketten in den Reaktionansätzen wurden durch Erhitzen in Reduktionsmittelhaltigem Probenpuffer gespalten. Spuren 5-7: Die Disulfidbrücken der Fab Ketten in den Reaktionansätzen bleiben durch Behandlung in Probenpuffer ohne Reduktionsmittel erhalten. Spuren 1 und 5: Koexpression von leichter und schwerer Kette von Anti-Hühnereiweis-Lysozym Fab Antikörperfragment, Spuren 2 und 6: Kontrollansatz mit Anti-Lysozym Einzelstrangantikörper ohne Signalpeptid synthetisiert in Gegenwart von 0,5 *µ*M Proteindisulfidisomerase (Takara), Spuren 3 und 7: Koexpression von leichter und schwerer Kette von Anti-humanes CD4 Fab Antikörperfragment, Spur 4: leer

### Beispiel 3

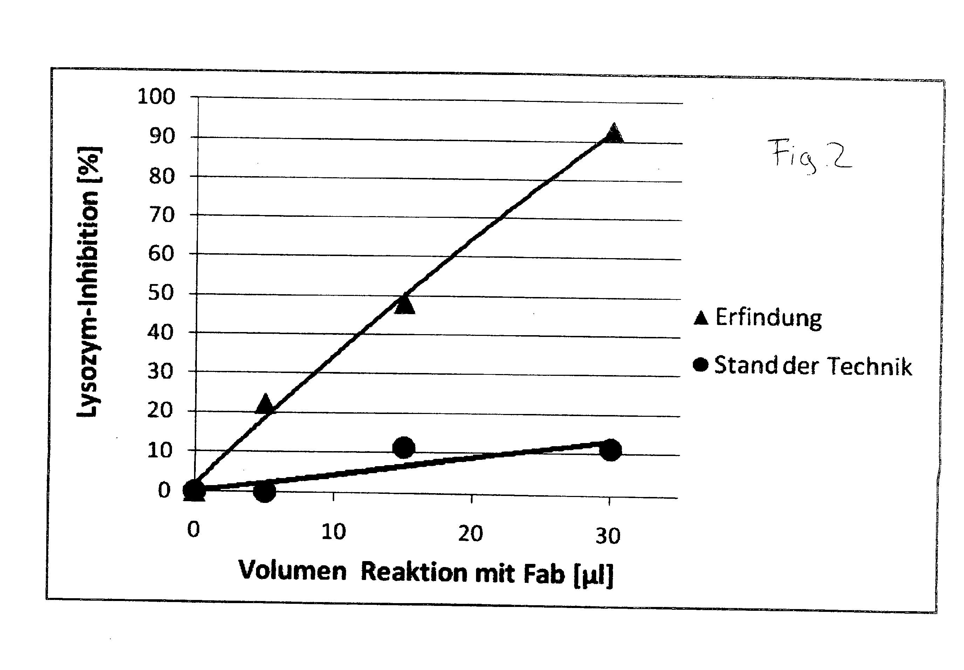

Ein Anti-Hühnereiweis-Lysozym Fab Antikörperfragment wird wie in Ausführungsbeispiel 1 beschrieben synthetisiert. Als Matrizen werden die Plasmide pIX5.0-Mel-LaLys und pIX5.0-HaLys, welche gemäß der hier beschriebenen Erfindung für ein Signalpeptid am N-Terminus der Fab Ketten kodieren, eingesetzt. Die für die Ausführung des Stands der Technik eingesetzten Plasmide, welche für dieselben Proteine kodieren wie die pIX5.0 Plasmidvarianten, jedoch keine Sequenz kodierend für Signalpeptid aufweisen, sind pIX3.0-LaLys und pIX3.0a-HaLys. Für die Bestimmung der Fab Aktivität wird Hühnereiweis-Lysozym (Sigma-Aldrich) mit einer spezifischen Aktivität von 56400 U/mg mit einem Aliquot des Reaktionsansatzes mit dem synthetisiertem Fab und parallel mit einem Aliquot aus einem Kontrollansatz inkubiert und die spezifische Inhibition der Lysozym-abhängigen Lyse von Micrococcus lysodeicticus Zellen wie in der Literaturstelle Merk H, Stiege W, Tsumoto K, Kumagai I, Erdmann VA (1999) Cell-free Expression of two Single-Chain Monoclonal Antibodies against Lysozyme: Effect of Domain Arrangement on the Expression. J Biochem 125:328-333 beschrieben bestimmt. Zum Vergleich wird parallel Anti-Hühnereiweis-Lysozym Fab Antikörperfragment gemäß dem Stand der Technik mit dem EasyXpress Insect Kit II (Qiagen) nach Herstellerangaben synthetisiert und ebenfalls für die Aktivitätsbestimmung eingesetzt. Das Ergebnis des Vergleichstests ist in Abbildung 2 dargestellt. Anti-Hühnereiweis-Lysozym Fab Antikörperfragment wurde zellfrei a) nach dem Stand der Technik und b) gemäß der hier beschriebenen Erfindung synthetisiert. Jeweils 5, 15 und 30 *µ*l Reaktionsansatz mit synthetisiertem Anti-Hühnereiweis-Lysozym Fab Antikörperfragment aus den verschiedenen Syntheseverfahren sowie jeweils 30 *µ*l Kontrollreaktionsansatz mit synthetisiertem Erythropoietin wurden mit 20 ng Hühnereiweis-Lysozym inkubiert und die spezifische Inhibition von Lysozym detektiert. Werte für unspezifische Inhibition, welche aus der Wirkung des jeweiligen Kontrollreaktionsansatzes hervorgehen, wurden von den Messwerten abgezogen.

Fab, welches nach dem hier beschriebenen Verfahren synthetisiert wird, zeigt eine um etwa eine Größenordnung höhere Aktivität im Vergleich zu einem Ansatz nach dem Stand der Technik.

### Beispiel 4

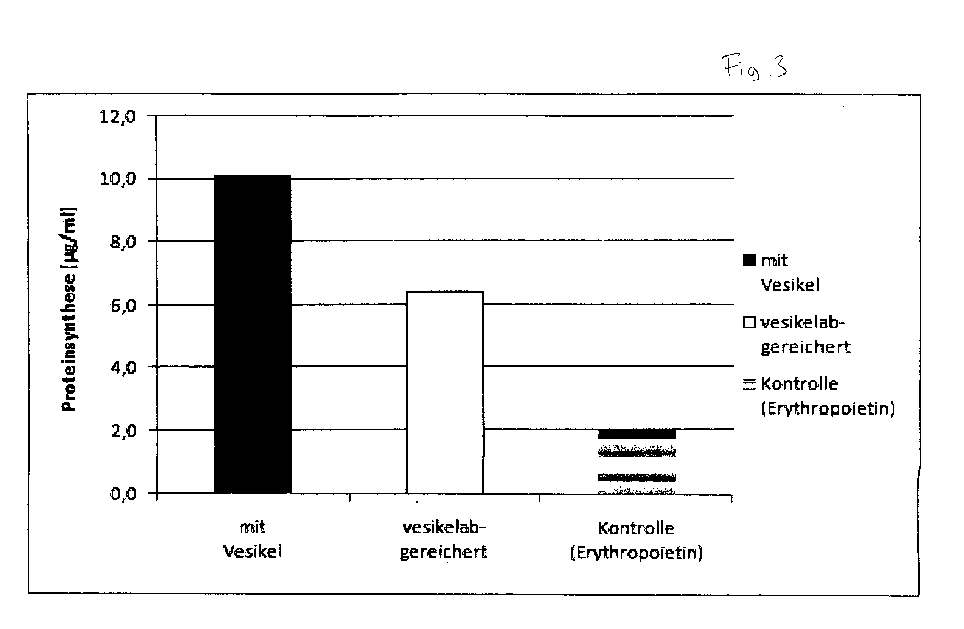

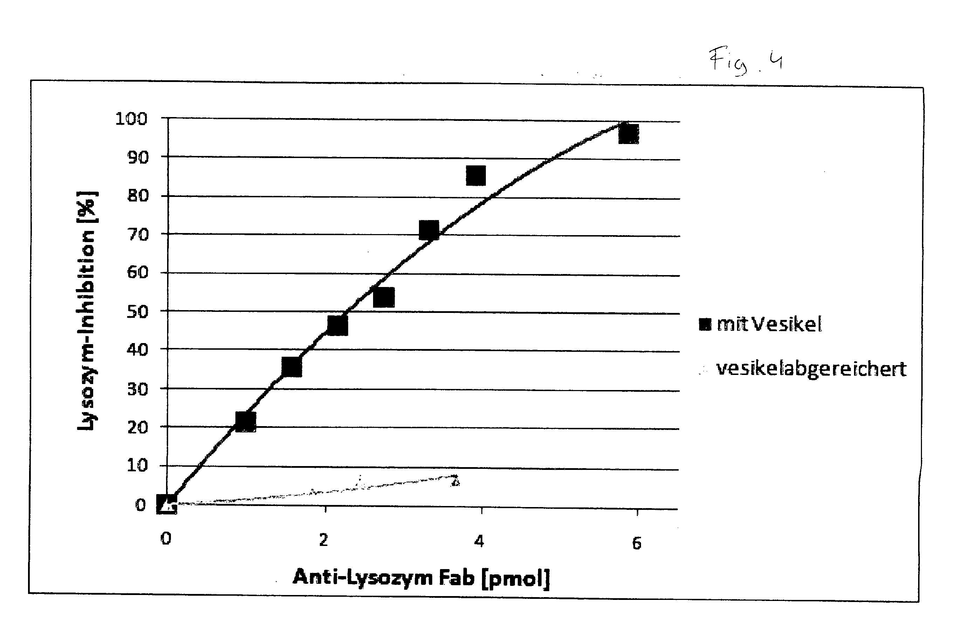

Ein Anti-Hühnereiweis-Lysozym Fab Antikörperfragment wird wie in Ausführungsbeispiel 1 beschrieben synthetisiert mit dem Unterschied, dass aus dem nukleasebehandelten Lysat vor der Synthesereaktion mikrosomale, dem Endoplasmatischen Reticulum entstammende Vesikel weitgehend entfern wurden. Hierzu wird das Lysat 20 Minuten bei 16000 x g und 4°C zentrifugiert. Der von den pelletierten Vesikel befreite Zentrifugationsüberstand des nukleasebehandelten Lysats wird dann für die Syntheseraktion von Fab eingesetzt. Parallel hierzu wird die Synthese von Fab gemäß der Erfindung mit Vesikel enthaltendem Lysat wie in Ausführungsbeispiel 2 beschrieben synthetisiert. Ausbeute und Aktivität der synthetisierten Proteine sind in den Abbildungen 3 und 4 beschrieben.

Abb. 3 zeigt die Bestimmung der Syntheseseausbeute von Fab synthetisiert gemäß der Erfindung und vergleichend mit vesikelabgereichertem Lysat. Ausbeuten der neu synthetisierten, radioaktiv markierten Proteine wurden anhand von Szintillationsmessung des in heißer Trichloressigsäure unlöslichen Matrials in 5 *µ*l Aliquots der Reaktionsansätze bestimmt. Abb. 4 zeigt die Aktivitätsbestimmung von Fab synthetisiert gemäß der Erfindung und mit vesikelabgereichertem Lysat. Die Aktivität von Anti-Hühnereiweis-Lysozym Fab Antikörperfragment wurde analog zum Ausführungsbeispiel 3 bestimmt. Dargestellt ist das jeweilige Ausmaß der Lysozym-Inhibition in Abhängigkeit der eingesetzten Fab Stoffmenge, welche entsprechend Abbildung 3 über Radioaktivitätsmessung bestimmt wurde.

Fab, welches gemäß der hier beschriebenen Erfindung synthetisiert wird, zeigt eine um etwa eine Größenordnung höhere Aktivität im Vergleich zu einem Ansatz, welcher nicht die Translokation von Fab in mikrosomale Vesikel und damit nur einen Teil des Verfahrens dieser Erfindung beinhaltet.

### Beispiel 5

Folgend werden Vektorkarten und Sequenzen der für die zellfreie Proteinsynthese verwendeten DNA-Matrizen dargestellt und beschrieben.

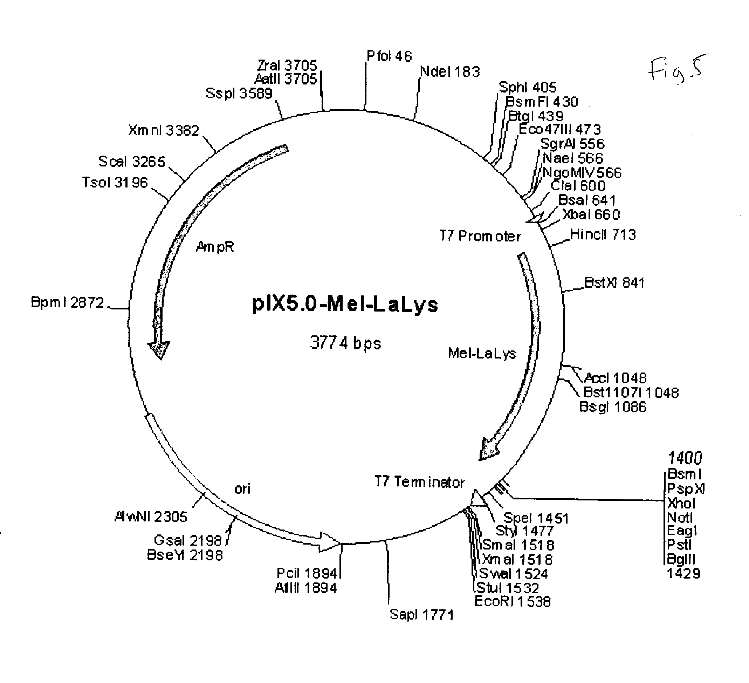

Abb. 5 zeigt das Plasmid pIX5.0-Mel-LaLys (Plasmid DNA) mit den folgenden Squenzdetails:

| Typ | Start | Ende | Name |
|---|---|---|---|
| Region | 622 | 638 | T7 Transkriptionspromoter |
| Gen | 701 | 1408 | Mel-LaLys, leichte Kette |
| anti-Lysozym Fab mit Melittin-Signalpeptid | | | |
| Region | 1475 | 1513 | T7 Transkriptionsterminator |
| Region | 2569 | 1896 | komplementär, |
| Replikationsursprung (ori) | | | |
| Gen | 3574 | 2714 | komplementär, Ampicillin |
| Resistenzgen (β-Lactamase) | | | |

TCGCGCGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCGGGTGTT
GGCGGGTGTCGGGGCTGGCTTAACTATGCGGCATCAGAGCAGATTGTACTGAGAGTGCACC
ATATGCGGTGTGAAATACCGCACAGATGCGTAAGGAGAAAATACCGCATCAGGCGCCATTC
GCCATTCAGGCTGCGCAACTGTTGGGAAGGGCGATCGGTGCGGGCCTCTTCGCTATTACGC
CAGCTGGCGAAAGGGGGATGTGCTGCAAGGCGATTAAGTTGGGTAACGCCAGGGTTTTCCC
AGTCACGACGTTGTAAAACGACGGCCAGTGCCAAGCTTGCATGCAAGGAGATGGCGCCCAA
CAGTCCCCCGGCCACGGGGCCTGCCACCATACCCACGCCGAAACAAGCGCTCATGAGCCCG
AAGTGGCGAGCCCGATCTTCCCCATCGGTGATGTCGGCGATATAGGCGCCAGCAACCGCAC
CTGTGGCGCCGGTGATGCCGGCCACGATGCGTCCGGCGTAGAGGATCGAGATCGATCTCGA
TCCCGCGAAATTAATACGACTCACTATAGGGAGACCACAACGGTTTCCCTCTAGAAATAAT
TTTGTTTAACTTTAAGAAGGAGATATACCATGAAATTCTTAGTCAACGTTGCCCTGGTTTT
TATGGTGGTGTATATTAGCTATATTTATGCCGATATTGTGCTGACCCAGAGTCCGGCAACC
CTGAGCGTTACACCGGGTAATAGCGTTAGCCTGAGCTGTCGTGCAAGCCAGAGCATTGGTA
ATAATCTGCATTGGTATCAGCAGAAAAGCCATGAAAGTCCGCGTCTGCTGATTAAATATGC
AAGCCAGTCAATTAGCGGTATTCCGAGCCGTTTTAGCGGTAGCGGTAGTGGCACCGATTTT
ACCCTGAGCATTAATAGCGTTGAAACCGAAGATTTTGGCATGTATTTTTGCCAGCAGAGCA
ATAGCTGGCCGTATACCTTTGGTGGTGGCACCAAACTGGAAATTAAACGTGCAGATGCAGC
ACCGACCGTTAGCATTTTTCCGCCGAGCAGCGAACAGCTGACCAGCGGTGGTGCAAGCGTT
GTTTGTTTTCTGAATAACTTTTATCCGAAAGATATCAATGTGAAATGGAAAATTGATGGCA
GCGAACGTCAGAATGGTGTTCTGAATAGCTGGACCGATCAGGATAGCAAAGATAGCACCTA
TAGCATGAGCAGCACCCTGACCCTGACCAAAGATGAATATGAACGCCATAATAGCTATACC

TGTGAAGCAACCCATAAAACCAGCACCAGCCCGATTGTTAAAAGCTTTAATCGCAATGAAT
GCTAATAACTCGAGCGGCCGCCTGCAGATCTAAATAATAAGTAATTAACTAGTGAGCAATA
ACTAGCATAACCCCTTGGGGCCTCTAAACGGGTCTTGAGGGGTTTTTTGATATCCCGGAT
TTAAATAGGCCTGAATTCGTAATCATGGTCATAGCTGTTTCCTGTGTGAAATTGTTATCCG
CTCACAATTCCACACAACATACGAGCCGGAAGCATAAAGTGTAAAGCCTGGGGTGCCTAAT
GAGTGAGCTAACTCACATTAATTGCGTTGCGCTCACTGCCCGCTTTCCAGTCGGGAAACCT
GTCGTGCCAGCTGCATTAATGAATCGGCCAACGCGCGGGGAGAGGCGGTTTGCGTATTGGG
CGCTCTTCCGCTTCCTCGCTCACTGACTCGCTGCGCTCGGTCGTTCGGCTGCGGCGAGCGG
TATCAGCTCACTCAAAGGCGGTAATACGGTTATCCACAGAATCAGGGGATAACGCAGGAAA
GAACATGTGAGCAAAAGGCCAGCAAAAGGCCAGGAACCGTAAAAAGGCCGCGTTGCTGGCG
TTTTTCCATAGGCTCCGCCCCCCTGACGAGCATCACAAAAATCGACGCTCAAGTCAGAGGT
GGCGAAACCCGACAGGACTATAAAGATACCAGGCGTTTCCCCCTGGAAGCTCCCTCGTGCG
CTCTCCTGTTCCGACCCTGCCGCTTACCGGATACCTGTCCGCCTTTCTCCCTTCGGGAAGC
GTGGCGCTTTCTCATAGCTCACGCTGTAGGTATCTCAGTTCGGTGTAGGTCGTTCGCTCCA
AGCTGGGCTGTGTGCACGAACCCCCCGTTCAGCCCGACCGCTGCGCCTTATCCGGTAACTA
TCGTCTTGAGTCCAACCCGGTAAGACACGACTTATCGCCACTGGCAGCAGCCACTGGTAAC
AGGATTAGCAGAGCGAGGTATGTAGGCGGTGCTACAGAGTTCTTGAAGTGGTGGCCTAACT
ACGGCTACACTAGAAGGACAGTATTTGGTATCTGCGCTCTGCTGAAGCCAGTTACCTTCGG
AAAAAGAGTTGGTAGCTCTTGATCCGGCAAACAAACCACCGCTGGTAGCGGTGGTTTTTTT
GTTTGCAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGAGATT
ATCAAAAAGGATCTTCACCTAGATCCTTTTAAATTAAAAATGAAGTTTTAAATCAATCTAA
AGTATATATGAGTAAACTTGGTCTGACAGTTACCAATGCTTAATCAGTGAGGCACCTATCT
CAGCGATCTGTCTATTTCGTTCATCCATAGTTGCCTGACTCCCCGTCGTGTAGATAACTAC
GATACGGGAGGGCTTACCATCTGGCCCCAGTGCTGCAATGATACCGCGAGATCCACGCTCA
CCGGCTCCAGATTTATCAGCAATAAACCAGCCAGCCGGAAGGGCCGAGCGCAGAAGTGGTC
CTGCAACTTTATCCGCCTCCATCCAGTCTATTAATTGTTGCCGGGAAGCTAGAGTAAGTAG
TTCGCCAGTTAATAGTTTGCGCAACGTTGTTGCCATTGCTACAGGCATCGTGGTGTCACGC
TCGTCGTTTGGTATGGCTTCATTCAGCTCCGGTTCCCAACGATCAAGGCGAGTTACATGAT
CCCCCATGTTGTGCAAAAAAGCGGTTAGCTCCTTCGGTCCTCCGATCGTTGTCAGAAGTAA
GTTGGCCGCAGTGTTATCACTCATGGTTATGGCAGCACTGCATAATTCTCTTACTGTCATG
CCATCCGTAAGATGCTTTTCTGTGACTGGTGAGTACTCAACCAAGTCATTCTGAGAATAGT
GTATGCGGCGACCGAGTTGCTCTTGCCCGGCGTCAATACGGGATAATACCGCGCCACATAG
CAGAACTTTAAAAGTGCTCATCATTGGAAAACGTTCTTCGGGGCGAAAACTCTCAAGGATC

TTACCGCTGTTGAGATCCAGTTCGATGTAACCCACTCGTGCACCCAACTGATCTTCAGCAT
CTTTTACTTTCACCAGCGTTTCTGGGTGAGCAAAAACAGGAAGGCAAAATGCCGCAAAAAA
GGGAATAAGGGCGACACGGAAATGTTGAATACTCATACTCTTCCTTTTTCAATATTATTGA
AGCATTTATCAGGGTTATTGTCTCATGAGCGGATACATATTTGAATGTATTTAGAAAAATA
AACAAATAGGGGTTCCGCGCACATTTCCCCGAAAAGTGCCACCTGACGTCTAAGAAACCAT
TATTATCATGACATTAACCTATAAAAATAGGCGTATCACGAGGCCCTTTCGTC

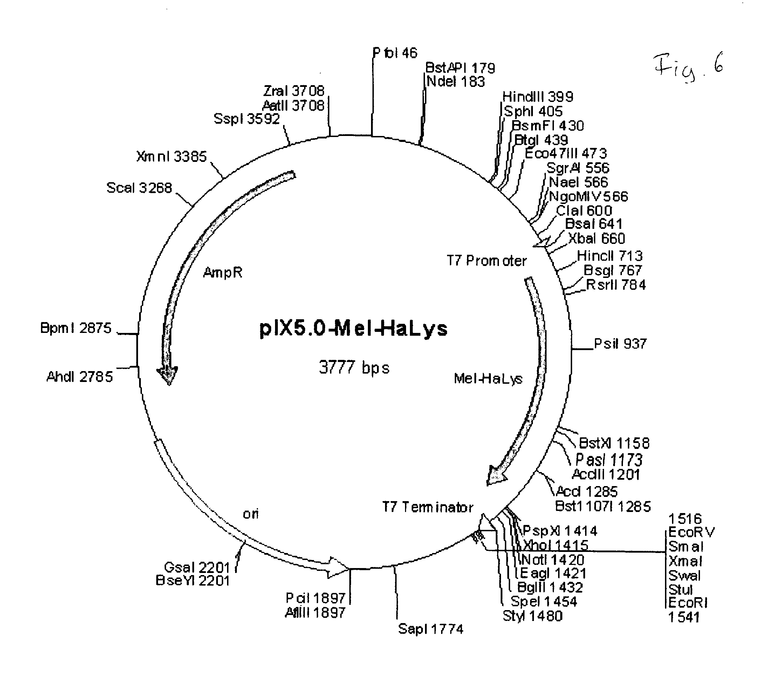

Abb. 6 zeigt das Plasmid pIX5.0-Mel-HaLys (Plasmid DNA) mit den folgenden Sequenzdetails:

| Typ | Start | Ende | Name |
|---|---|---|---|
| Region | 622 | 638 | T7 Transkriptionspromoter |
| Gen | 701 | 1411 | Mel-HaLys, leichte Kette |
| anti-Lysozym Fab mit Melittin-Signalpeptid | | | |
| Region | 1478 | 1516 | T7 Transkriptionsterminator |
| Region | 2572 | 1899 | komplementär, |
| Replikationsursprung (ori) | | | |
| Gen | 3577 | 2717 | komplementär, Ampicillin |
| Resistenzgen (β-Lactamase) | | | |

TCGCGCGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCGGGTGTT
GGCGGGTGTCGGGGCTGGCTTAACTATGCGGCATCAGAGCAGATTGTACTGAGAGTGCACC
ATATGCGGTGTGAAATACCGCACAGATGCGTAAGGAGAAAATACCGCATCAGGCGCCATTC
GCCATTCAGGCTGCGCAACTGTTGGGAAGGGCGATCGGTGCGGGCCTCTTCGCTATTACGC
CAGCTGGCGAAAGGGGGATGTGCTGCAAGGCGATTAAGTTGGGTAACGCCAGGGTTTTCCC
AGTCACGACGTTGTAAAACGACGGCCAGTGCCAAGCTTGCATGCAAGGAGATGGCGCCCAA
CAGTCCCCCGGCCACGGGGCCTGCCACCATACCCACGCCGAAACAAGCGCTCATGAGCCCG
AAGTGGCGAGCCCGATCTTCCCCATCGGTGATGTCGGCGATATAGGCGCCAGCAACCGCAC
CTGTGGCGCCGGTGATGCCGGCCACGATGCGTCCGGCGTAGAGGATCGAGATCGATCTCGA
TCCCGCGAAATTAATACGACTCACTATAGGGAGACCACAACGGTTTCCCTCTAGAAATAAT
TTTGTTTAACTTTAAGAAGGAGATATACCATGAAATTCTTAGTCAACGTTGCCCTGGTTTT
TATGGTGGTGTATATTAGCTATATTTATGCCGATGTGCAGCTGCAGGAAAGCGGTCCGAGC
CTGGTTAAACCGAGCCAGACCCTGAGCCTGACCTGTAGCGTTACCGGTGATAGCATTACCA

GCGATTATTGGAGCTGGATTCGTAAATTTCCGGGTAATCGTCTGGAATATATGGGTTATGT
TAGCTATAGCGGCAGCACCTATTATAATCCGAGCCTGAAAAGCCGTATTAGCATTACCCGT
GATACCAGCAAAAATCAGTATTATCTGGATCTGAATAGCGTGACCACCGAAGATACCGCAA
CCTATTACTGTGCAAATTGGGATGGTGATTATTGGGGTCAGGGCACCCTGGTTACCGTTAG
CGCAGCAAAAACCACACCGCCGAGCGTTTATCCGCTGGCACCGGGTAGCGCAGCACAGACC
AATAGCATGGTTACCCTGGGTTGTCTGGTGAAAGGTTATTTCCGGAACCGGTTACCGTTA
CCTGGAATAGCGGTAGCCTGAGCAGCGGTGTTCATACCTTTCCGGCAGTTCTGCAGAGCGA
TCTGTATACCCTGAGCAGCAGCGTTACCGTTCCGAGCAGTCCGCGTCCGAGCGAAACCGTT
ACCTGTAATGTTGCACATCCGGCAAGCAGCACCAAAGTTGATAAAAAAATTGTTCCGCGTG
ATTGCTAATAACTCGAGCGGCCGCCTGCAGATCTAAATAATAAGTAATTAACTAGTGAGCA
ATAACTAGCATAACCCCTTGGGGCCTCTAAACGGGTCTTGAGGGGTTTTTTGATATCCCGG
GATTTAAATAGGCCTGAATTCGTAATCATGGTCATAGCTGTTTCCTGTGTGAAATTGTTAT
CCGCTCACAATTCCACACAACATACGAGCCGGAAGCATAAAGTGTAAAGCCTGGGGTGCCT
AATGAGTGAGCTAACTCACATTAATTGCGTTGCGCTCACTGCCCGCTTTCCAGTCGGGAAA
CCTGTCGTGCCAGCTGCATTAATGAATCGGCCAACGCGCGGGGAGAGGCGGTTTGCGTATT
GGGCGCTCTTCCGCTTCCTCGCTCACTGACTCGCTGCGCTCGGTCGTTCGGCTGCGGCGAG
CGGTATCAGCTCACTCAAAGGCGGTAATACGGTTATCCACAGAATCAGGGGATAACGCAGG
AAAGAACATGTGAGCAAAAGGCCAGCAAAAGGCCAGGAACCGTAAAAAGGCCGCGTTGCTG
GCGTTTTTCCATAGGCTCCGCCCCCCTGACGAGCATCACAAAAATCGACGCTCAAGTCAGA
GGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGTAGGTCGTTCGCT
CCAAGCTGGGCTGTGTGCACGAACCCCCCGTTCAGCCCGACCGCTGCGCCTTATCCGGTAA
CTATCGTCTTGAGTCCAACCCGGTAAGACACGACTTATCGCCACTGGCAGCAGCCACTGGT
AACAGGATTAGCAGAGCGAGGTATGTAGGCGGTGCTACAGAGTTCTTGAAGTGGTGGCCTA
ACTACGGCTACACTAGAAGGACAGTATTTGGTATCTGCGCTCTGCTGAAGCCAGTTACCTT
CGGAAAAAGAGTTGGTAGCTCTTGATCCGGCAAACAAACCACCGCTGGTAGCGGTGGTTTT
TTTGTTTGCAAGCAGCAGATTACGCGCAGAAAAAAAGGATCTCAAGAAGATCCTTTGATCT
TTTCTACGGGGTCTGACGCTCAGTGGAACGAAAACTCACGTTAAGGGATTTTGGTCATGAG
ATTATCAAAAAGGATCTTCACCTAGATCCTTTTAAATTAAAAATGAAGTTTTAAATCAATC
TAAAGTATATATGAGTAAACTTGGTCTGACAGTTACCAATGCTTAATCAGTGAGGCACCTA
TCTCAGCGATCTGTCTATTTCGTTCATCCATAGTTGCCTGACTCCCCGTCGTGTAGATAAC
TACGATACGGGAGGGCTTACCATCTGGCCCCAGTGCTGCAATGATACCGCGAGATCCACGC
TCACCGGCTCCAGATTTATCAGCAATAAACCAGCCAGCCGGAAGGGCCGAGCGCAGAAGTG

GTCCTGCAACTTTATCCGCCTCCATCCAGTCTATTAATTGTTGCCGGGAAGCTAGAGTAAG
TAGTTCGCCAGTTAATAGTTTGCGCAACGTTGTTGCCATTGCTACAGGCATCGTGGTGTCA
CGCTCGTCGTTTGGTATGGCTTCATTCAGCTCCGGTTCCCAACGATCAAGGCGAGTTACAT
GATCCCCCATGTTGTGCAAAAAAGCGGTTAGCTCCTTCGGTCCTCCGATCGTTGTCAGAAG
TAAGTTGGCCGCAGTGTTATCACTCATGGTTATGGCAGCACTGCATAATTCTCTTACTGTC
ATGCCATCCGTAAGATGCTTTTCTGTGACTGGTGAGTACTCAACCAAGTCATTCTGAGAAT
AGTGTATGCGGCGACCGAGTTGCTCTTGCCCGGCGTCAATACGGGATAATACCGCGCCACA
TAGCAGAACTTTAAAAGTGCTCATCATTGGAAAACGTTCTTCGGGGCGAAAACTCTCAAGG
ATCTTACCGCTGTTGAGATCCAGTTCGATGTAACCCACTCGTGCACCCAACTGATCTTCAG
CATCTTTTACTTTCACCAGCGTTTCTGGGTGAGCAAAAACAGGAAGGCAAAATGCCGCAAA
AAAGGGAATAAGGGCGACACGGAAATGTTGAATACTCATACTCTTCCTTTTTCAATATTAT
TGAAGCATTTATCAGGGTTATTGTCTCATGAGCGGATACATATTTGAATGTATTTAGAAAA
ATAAACAAATAGGGGTTCCGCGCACATTTCCCCGAAAAGTGCCACCTGACGTCTAAGAAAC
CATTATTATCATGACATTAACCTATAAAAATAGGCGTATCACGAGGCCCTTTCGTC

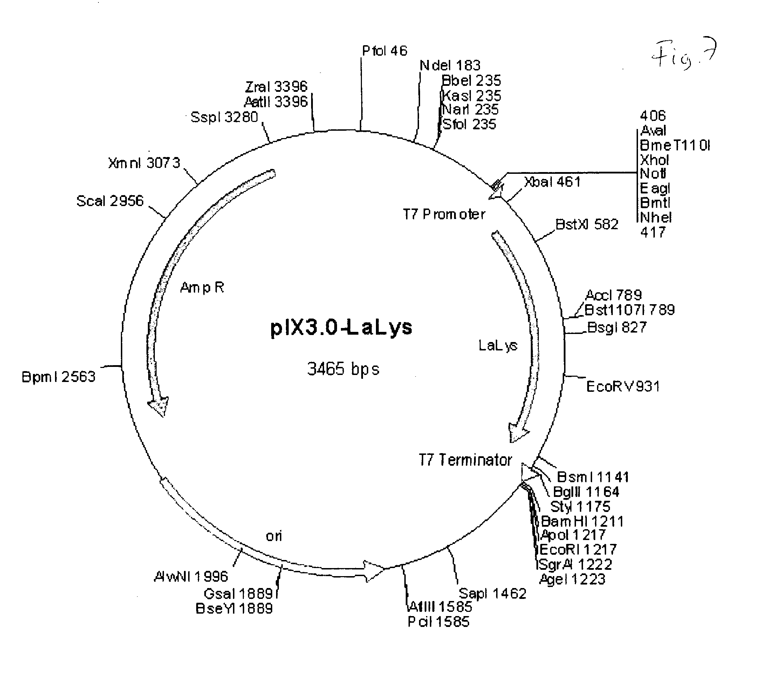

Abb. 7 zeigt das Plasmid pIX3.0-LaLys (Plasmid DNA) mit den folgenden Sequenzdetails:

| Typ | Start | Ende | Name |
|---|---|---|---|
| Region | 423 | 439 | T7 Transkriptionspromoter |
| Gen | 502 | 1149 | HaLys, leichte Kette |
| anti-Lysozym Fab | | | |
| Region | 1173 | 1211 | T7 Transkriptionsterminator |
| Region | 2260 | 1627 | komplementär, |
| Replikationsursprung (ori) | | | |
| Gen | 3265 | 2405 | komplementär, Ampicillin |
| Resistenzgen (β-Lactamase) | | | |

TCGCGCGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCGGGTGTT
GGCGGGTGTCGGGGCTGGCTTAACTATGCGGCATCAGAGCAGATTGTACTGAGAGTGCACC
ATATGCGGTGTGAAATACCGCACAGATGCGTAAGGAGAAAATACCGCATCAGGCGCCATTC

GCCATTCAGGCTGCGCAACTGTTGGGAAGGGCGATCGGTGCGGGCCTCTTCGCTATTACGC
CAGCTGGCGAAAGGGGGATGTGCTGCAAGGCGATTAAGTTGGGTAACGCCAGGGTTTTCCC
AGTCACGACGTTGTAAAACGACGGCCAGTGCCAAGCTTTCTCGAGCGGCCGCTAGCTAATA
CGACTCACTATAGGGAGACCACAACGGTTTCCCTCTAGAAATAATTTTGTTTAACTTTAAG
AAGGAGATAAACAATGGATATTGTGCTGACCCAGAGTCCGGCAACCCTGAGCGTTACACCG
GGTAATAGCGTTAGCCTGAGCTGTCGTGCAAGCCAGAGCATTGGTAATAATCTGCATTGGT
ATCAGCAGAAAAGCCATGAAAGTCCGCGTCTGCTGATTAAATATGCAAGCCAGTCAATTAG
CGGTATTCCGAGCCGTTTTAGCGGTAGCGGTAGTGGCACCGATTTTACCCTGAGCATTAAT
AGCGTTGAAACCGAAGATTTTGGCATGTATTTTTGCCAGCAGAGCAATAGCTGGCCGTATA
CCTTTGGTGGTGGCACCAAACTGGAAATTAAACGTGCAGATGCAGCACCGACCGTTAGCAT
TTTTCCGCCGAGCAGCGAACAGCTGACCAGCGGTGGTGCAAGCGTTGTTTGTTTTCTGAAT
AACTTTTATCCGAAAGATATCAATGTGAAATGGAAAATTGATGGCAGCGAACGTCAGAATG
GTGTTCTGAATAGCTGGACCGATCAGGATAGCAAAGATAGCACCTATAGCATGAGCAGCAC
CCTGACCCTGACCAAAGATGAATATGAACGCCATAATAGCTATACCTGTGAAGCAACCCAT
AAAACCAGCACCAGCCCGATTGTTAAAAGCTTTAATCGCAATGAATGCTAATAACTAACTA
ACCAAGATCTGTACCCCTTGGGGCCTCTAAACGGGTCTTGAGGGGTTTTTTGGATCCGAAT
TCACCGGTGCAATTCGTAATCATGTCATAGCTGTTTCCTGTGTGAAATTGTTATCCGCTCA
CAATTCCACACAACATACGAGCCGGAAGCATAAAGTGTAAAGCCTGGGGTGCCTAATGAGT
GAGCTAACTCACATTAATTGCGTTGCGCTCACTGCCCGCTTTCCAGTCGGGAAACCTGTCG
TGCCAGCTGCATTAATGAATCGGCCAACGCGCGGGGAGAGGCGGTTTGCGTATTGGGCGCT
CTTCCGCTTCCTCGCTCACTGACTCGCTGCGCTCGGTCGTTCGGCTGCGGCGAGCGGTATC
AGCTCACTCAAAGGCGGTAATACGGTTATCCACAGAATCAGGGGATAACGCAGGAAAGAAC
ATGTGAGCAAAAGGCCAGCAAAAGGCCAGGAACCGTAAAAAGGCCGCGTTGCTGGCGTTTT
TCCATAGGCTCCGCCCCCCTGACGAGCATCACAAAAATCGACGCTCAAGTCAGAGGTGGCG
AAACCCGACAGGACTATAAAGATACCAGGCGTTTCCCCCTGGAAGCTCCCTCGTGCGCTCT
CCTGTTCCGACCCTGCCGCTTACCGGATACCTGTCCGCCTTTCTCCCTTCGGGAAGCGTGG
CGCTTTCTCATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGTAACTATCGT
CTTGAGTCCAACCCGGTAAGACACGACTTATCGCCACTGGCAGCAGCCACTGGTAACAGGA
TTAGCAGAGCGAGGTATGTAGGCGGTGCTACAGAGTTCTTGAAGTGGTGGCCTAACTACGG
CTACACTAGAAGAACAGTATTTGGTATCTGCGCTCTGCTGAAGCCAGTTACCTTCGGAAAA
AGAGTTGGTAGCTCTTGATCCGGCAAACAAACCACCGCTGGTAGCGGTGGTTTTTTTGTTT
GCAAGCAGCAGATTACGCGCAGAAAAAAAGGATCTCAAGAAGATCCTTTGATCTTTTCTAC
GGGGTCTGACGCTCAGTGGAACGAAAACTCACGTTAAGGGATTTTGGTCATGAGATTATCA

AAAAGGATCTTCACCTAGATCCTTTTAAATTAAAAATGAAGTTTTAAATCAATCTAAAGTA
TATATGAGTAAACTTGGTCTGACAGTTACCAATGCTTAATCAGTGAGGCACCTATCTCAGC
GATCTGTCTATTTCGTTCATCCATAGTTGCCTGACTCCCCGTCGTGTAGATAACTACGATA
CGGGAGGGCTTACCATCTGGCCCCAGTGCTGCAATGATACCGCGAGACCCACGCTCACCGG
CTCCAGATTTATCAGCAATAAACCAGCCAGCCGGAAGGGCCGAGCGCAGAAGTGGTCCTGC
AACTTTATCCGCCTCCATCCAGTCTATTAATTGTTGCCGGGAAGCTAGAGTAAGTAGTTCG
CCAGTTAATAGTTTGCGCAACGTTGTTGCCATTGCTACAGGCATCGTGGTGTCACGCTCGT
CGTTTGGTATGGCTTCATTCAGCTCCGGTTCCCAACGATCAAGGCGAGTTACATGATCCCC
CATGTTGTGCAAAAAAGCGGTTAGCTCCTTCGGTCCTCCGATCGTTGTCAGAAGTAAGTTG
GCCGCAGTGTTATCACTCATGGTTATGGCAGCACTGCATAATTCTCTTACTGTCATGCCAT
CCGTAAGATGCTTTTCTGTGACTGGTGAGTACTCAACCAAGTCATTCTGAGAATAGTGTAT
GCGGCGACCGAGTTGCTCTTGCCCGGCGTCAATACGGGATAATACCGCGCCACATAGCAGA
ACTTTAAAAGTGCTCATCATTGGAAAACGTTCTTCGGGGCGAAAACTCTCAAGGATCTTAC
CGCTGTTGAGATCCAGTTCGATGTAACCCACTCGTGCACCCAACTGATCTTCAGCATCTTT
TACTTTCACCAGCGTTTCTGGGTGAGCAAAAACAGGAAGGCAAAATGCCGCAAAAAAGGGA
ATAAGGGCGACACGGAAATGTTGAATACTCATACTCTTCCTTTTTCAATATTATTGAAGCA
TTTATCAGGGTTATTGTCTCATGAGCGGATACATATTTGAATGTATTTAGAAAAATAAACA
AATAGGGGTTCCGCGCACATTTCCCCGAAAAGTGCCACCTGACGTCTAAGAAACCATTATT
ATCATGACATTAACCTATAAAAATAGGCGTATCACGAGGCCCTTTCGTC

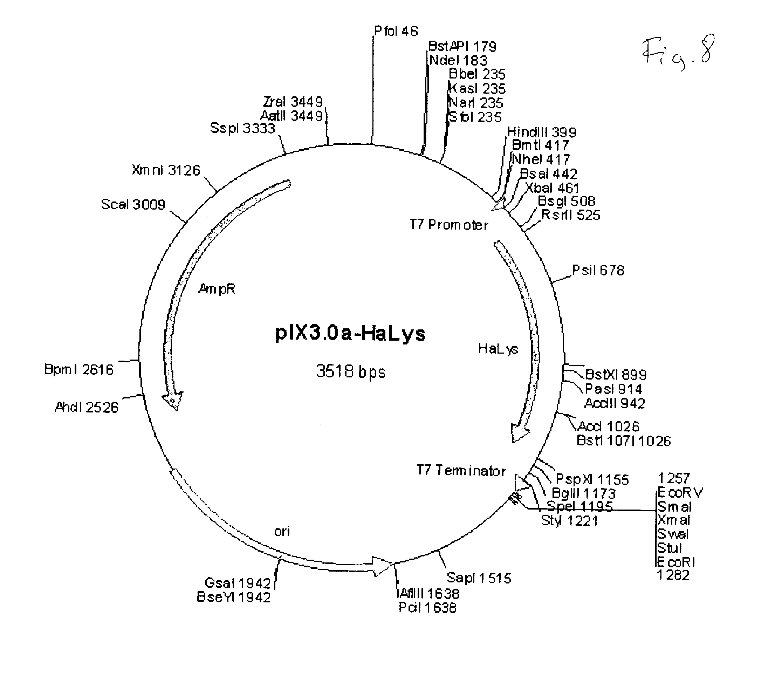

Abb. 8 zeigt das Plasmid pIX3.0a-HaLys (Plasmid DNA) mit den folgenden Sequenzdetails:

| Typ | Start | Ende | Name |
|---|---|---|---|
| Region | 423 | 439 | T7 Transkriptionspromoter |
| Gen | 502 | 1152 | HaLys, leichte Kette |
| anti-Lysozym Fab | | | |
| Region | 1219 | 1257 | T7 Transkriptionsterminator |
| Region | 2313 | 1640 | komplementär, |
| Replikationsursprung (ori) | | | |
| Gen | 3318 | 2458 | komplementär, Ampicillin |
| Resistenzgen (β-Lactamase) | | | |

TCGCGCGTTTCGGTGATGACGGTGAAAACCTCTGACACATGCAGCTCCCGGAGACGGTCAC
AGCTTGTCTGTAAGCGGATGCCGGGAGCAGACAAGCCCGTCAGGGCGCGTCAGCGGGTGTT
GGCGGGTGTCGGGGCTGGCTTAACTATGCGGCATCAGAGCAGATTGTACTGAGAGTGCACC
ATATGCGGTGTGAAATACCGCACAGATGCGTAAGGAGAAAATACCGCATCAGGCGCCATTC
GCCATTCAGGCTGCGCAACTGTTGGGAAGGGCGATCGGTGCGGGCCTCTTCGCTATTACGC
CAGCTGGCGAAAGGGGGATGTGCTGCAAGGCGATTAAGTTGGGTAACGCCAGGGTTTTCCC
AGTCACGACGTTGTAAAACGACGGCCAGTGCCAAGCTTTCTCGAGCGGCCGCTAGCTAATA
CGACTCACTATAGGGAGACCACAACGGTTTCCCTCTAGAAATAATTTTGTTTAACTTTAAG
AAGGAGATAAACAATGGATGTGCAGCTGCAGGAAAGCGGTCCGAGCCTGGTTAAACCGAGC
CAGACCCTGAGCCTGACCTGTAGCGTTACCGGTGATAGCATTACCAGCGATTATTGGAGCT
GGATTCGTAAATTTCCGGGTAATCGTCTGGAATATATGGGTTATGTTAGCTATAGCGGCAG
CACCTATTATAATCCGAGCCTGAAAAGCCGTATTAGCATTACCCGTGATACCAGCAAAAAT
CAGTATTATCTGGATCTGAATAGCGTGACCACCGAAGATACCGCAACCTATTACTGTGCAA
ATTGGGATGGTGATTATTGGGGTCAGGGCACCCTGGTTACCGTTAGCGCAGCAAAAACCAC
ACCGCCGAGCGTTTATCCGCTGGCACCGGGTAGCGCAGCACAGACCAATAGCATGGTTACC
CTGGGTTGTCTGGTGAAAGGTTATTTCCGGAACCGGTTACCGTTACCTGGAATAGCGGTA
GCCTGAGCAGCGGTGTTCATACCTTTCCGGCAGTTCTGCAGAGCGATCTGTATACCCTGAG
CAGCAGCGTTACCGTTCCGAGCAGTCCGCGTCCGAGCGAAACCGTTACCTGTAATGTTGCA
CATCCGGCAAGCAGCACCAAAGTTGATAAAAAAATTGTTCCGCGTGATTGCTAATAACTCG
AGCGGCCGCCTGCAGATCTAAATAATAAGTAATTAACTAGTGAGCAATAACTAGCATAACC

CCTTGGGGCCTCTAAACGGGTCTTGAGGGGTTTTTTGATATCCCGGATTTAAATAGGCCT
GAATTCGTAATCATGGTCATAGCTGTTTCCTGTGTGAAATTGTTATCCGCTCACAATTCCA
CACAACATACGAGCCGGAAGCATAAAGTGTAAAGCCTGGGGTGCCTAATGAGTGAGCTAAC
TCACATTAATTGCGTTGCGCTCACTGCCCGCTTTCCAGTCGGGAAACCTGTCGTGCCAGCT
GCATTAATGAATCGGCCAACGCGCGGGGAGAGGCGGTTTGCGTATTGGGCGCTCTTCCGCT
TCCTCGCTCACTGACTCGCTGCGCTCGGTCGTTCGGCTGCGGCGAGCGGTATCAGCTCACT
CAAAGGCGGTAATACGGTTATCCACAGAATCAGGGGATAACGCAGGAAAGAACATGTGAGC
AAAAGGCCAGCAAAAGGCCAGGAACCGTAAAAAGGCCGCGTTGCTGGCGTTTTTCCATAGG
CTCCGCCCCCCTGACGAGCATCACAAAAATCGACGCTCAAGTCAGAGGTGGCGAAACCCGA
CAGGACTATAAAGATACCAGGCGTTTCCCCCTGGAAGCTCCCTCGTGCGCTCTCCTGTTCC
GACCCTGCCGCTTACCGGATACCTGTCCGCCTTTCTCCCTTCGGGAAGCGTGGCGCTTTCT
CATAGCTCACGCTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAGCAGA
GCGAGGTATGTAGGCGGTGCTACAGAGTTCTTGAAGTGGTGGCCTAACTACGGCTACACTA
GAAGGACAGTATTTGGTATCTGCGCTCTGCTGAAGCCAGTTACCTTCGGAAAAAGAGTTGG
TAGCTCTTGATCCGGCAAACAAACCACCGCTGGTAGCGGTGGTTTTTTTGTTTGCAAGCAG
CAGATTACGCGCAGAAAAAAAGGATCTCAAGAAGATCCTTTGATCTTTTCTACGGGGTCTG
ACGCTCAGTGGAACGAAAACTCACGTTAAGGGATTTTGGTCATGAGATTATCAAAAAGGAT
CTTCACCTAGATCCTTTTAAATTAAAAATGAAGTTTTAAATCAATCTAAAGTATATATGAG
TAAACTTGGTCTGACAGTTACCAATGCTTAATCAGTGAGGCACCTATCTCAGCGATCTGTC
TATTTCGTTCATCCATAGTTGCCTGACTCCCCGTCGTGTAGATAACTACGATACGGGAGGG
CTTACCATCTGGCCCCAGTGCTGCAATGATACCGCGAGATCCACGCTCACCGGCTCCAGAT
TTATCAGCAATAAACCAGCCAGCCGGAAGGGCCGAGCGCAGAAGTGGTCCTGCAACTTTAT
CCGCCTCCATCCAGTCTATTAATTGTTGCCGGGAAGCTAGAGTAAGTAGTTCGCCAGTTAA
TAGTTTGCGCAACGTTGTTGCCATTGCTACAGGCATCGTGGTGTCACGCTCGTCGTTTGGT
ATGGCTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCCGCAGT
GTTATCACTCATGGTTATGGCAGCACTGCATAATTCTCTTACTGTCATGCCATCCGTAAGA
TGCTTTTCTGTGACTGGTGAGTACTCAACCAAGTCATTCTGAGAATAGTGTATGCGGCGAC
CGAGTTGCTCTTGCCCGGCGTCAATACGGGATAATACCGCGCCACATAGCAGAACTTTAAA
AGTGCTCATCATTGGAAAACGTTCTTCGGGGCGAAAACTCTCAAGGATCTTACCGCTGTTG
AGATCCAGTTCGATGTAACCCACTCGTGCACCCAACTGATCTTCAGCATCTTTTACTTTCA
CCAGCGTTTCTGGGTGAGCAAAAACAGGAAGGCAAAATGCCGCAAAAAAGGGAATAAGGGC

GACACGGAAATGTTGAATACTCATACTCTTCCTTTTTCAATATTATTGAAGCATTTATCAG
GGTTATTGTCTCATGAGCGGATACATATTTGAATGTATTTAGAAAAATAAACAAATAGGGG
TTCCGCGCACATTTCCCCGAAAAGTGCCACCTGACGTCTAAGAAACCATTATTATCATGAC
ATTAACCTATAAAAATAGGCGTATCACGAGGCCCTTTCGTC

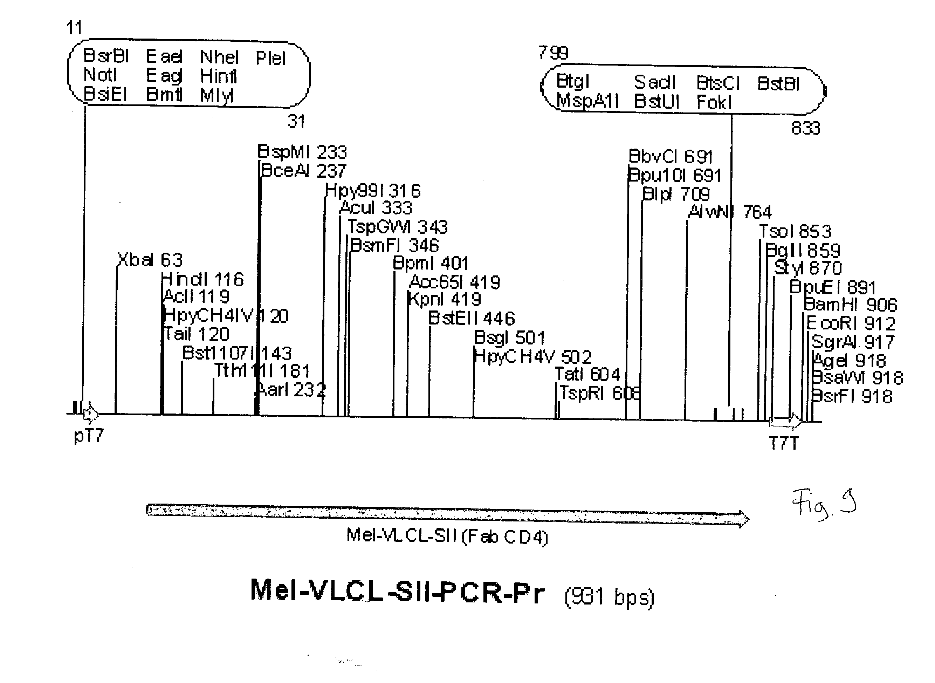

Abb. 9 zeigt die eingesetzte Matrize Mel-VLCL-SII, lineare, PCR-generierte DNA mit den folgenden Sequenzdetails:

| Typ | Start | Ende | Name |
|---|---|---|---|
| Region | 25 | 41 | T7 Transkriptionspromoter |
| Gen | 104 | 844 | Mel-LaLys, leichte Kette anti- |
| Lysozym Fab mit Melittin-Signalpeptid, C-terminaler Strep-Tag | | | |
| Region | 868 | 906 | T7 Transkriptionsterminator |

ATGATATCTCGAGCGGCCGCTAGCTAATACGACTCACTATAGGGAGACCACAACGGTTTCC
CTCTAGAAATAATTTTGTTTAACTTTAAGAAGGAGATAAACAATGAAATTCTTAGTCAACG
TTGCCCTTGTTTTTATGGTCGTATACATTTCTTACATCTATGCGGACGATATCCAGATGAC
CCAGTCTCCGGCTTCTCTGTCTGCTTCTGTTGGTGAAACCGTTACCTTCACCTGCCGTGCT
TCTGAAATGATCTACTCTTACCTGGCTTGGTATCAGCAGAAACAGGGTAAATCTCCGCAAC
TGCTGGTTCACGACGCTAAAACCCTGGCTGAAGGTGTTCCGTCCCGTTTCTCTGGTGGTGG
TTCTGGCACCCAGTTCTCTCTGAAAATCAACACCCTCCAGCCGGAAGACTTCGGTACCTAC
TACTGCCAGCACCACTACGGTAACCCGCCGACCTTCGGTGGTGGCACCAAACTCGAGATCA
AACGGGGGATCGCTGCACCATCTGTCTTCATCTTCCCGCCATCTGATGAGCAGTTGAAATC
TGGAACTGCCTCTGTTGTGTGCCTGCTGAATAACTTCTATCCCAGAGAGGCCAAAGTACAG
TGGAAGGTGGATAACGCCCTCCAATCGGGTAACTCCCAGGAGAGTGTCACAGAGCAGGACA
GCAAGGACAGCACCTACAGCCTCAGCAGCACCCTGACGCTGAGCAAAGCAGACTACGAGAA
ACACAAAGTCTACGCCTGCGAAGTCACCCATCAGGGCCTGAGTTCGCCCGTCACAAAGAGC
TTCAACCGCGGAGAGTGTTCTGCCTGGTCTCATCCGCAATTCGAAAAATAATAACTAACTA
ACCAAGATCTGTACCCCTTGGGGCCTCTAAACGGGTCTTGAGGGGTTTTTTGGATCCGAAT
TCACCGGTGATATCAT

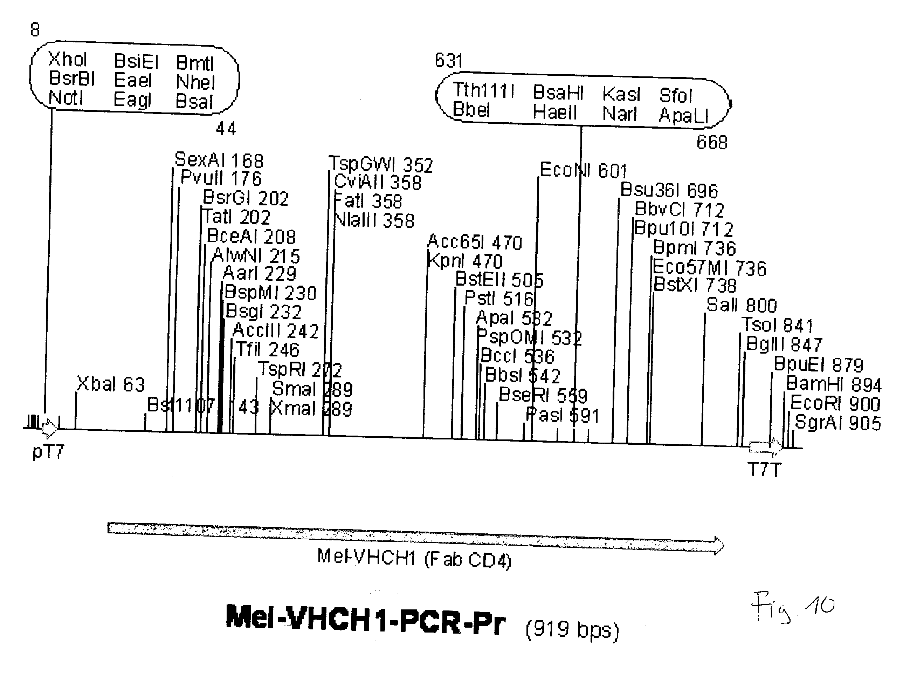

Abb. 10 zeigt die Matrize Mel-VHCHl, lineare, PCR-generierte DNA, mit den folgenden Sequenzdetails:

| Typ | Start | Ende | Name |
|---|---|---|---|
| Region | 25 | 41 | T7 Transkriptionspromoter |
| Gen | 104 | 832 | Mel-HaLys, schwere Kette |
| anti-Lysozym Fab mit Melittin-Signalpeptid | | | |
| Region | 856 | 894 | T7 Transkriptionsterminator |

ATGATATCTCGAGCGGCCGCTAGCTAATACGACTCACTATAGGGAGACCACAACGGTTTCC
CTCTAGAAATAATTTTGTTTAACTTTAAGAAGGAGATAAACAATGAAATTCTTAGTCAACG
TTGCCCTTGTTTTTATGGTCGTATACATTTCTTACATCTATGCGGACCAGGTTCAGCTGAA
ACAGTCTGGTCCGGGTCTTGTACAGCCGTCCCAGTCTCTGTCTATCACCTGCACCGTTTCC
GGATTCTCTCTGACCACCTTCGGTGTTCACTGGGTTCGTCAGTCCCCGGGTAAAGGTCTGG
AATGGCTGGGTGTTATCTGGCGTTCTGGTATCACCGACTACAACGTTCCGTTCATGTCTCG
TCTGTCTATCACCAAAGACAACTCTAAATCTCAGGTTTTCTTCAAACTGAACTCTCTGCAA
CCGGACGACACCGCTATCTACTACTGCGCTAAAAACGATCCGGGTACCGGTTTCGCTTACT
GGGGTCAGGGCACCCTGGTCACCGTTTCTGCAGGGAGCACCAAGGGCCCATCGGTCTTCCC
CCTGGCACCCTCCTCCAAGAGCACCTCTGGGGGCACAGCGGCCCTGGGCTGCCTGGTCAAG
GACTACTTCCCCGAACCGGTGACGGTGTCGTGGAACTCAGGCGCCCTGACCAGCGGCGTGC
ACACCTTCCCGGCTGTCCTACAGTCCTCAGGACTCTACTCCCTCAGCAGCGTGGTGACTGT
GCCCTCCAGCAGCTTGGGCACCCAGACCTACATCTGCAACGTTAATCACAAACCCAGCAAC
ACCAAGGTCGACAAGAAAGTTGAGCCCAAATCTTGCTAATAACTAACTAACCAAGATCTGT
ACCCCTTGGGGCCTCTAAACGGGTCTTGAGGGGTTTTTTGGATCCGAATTCACCGGTGATA
TCAT

## Patentansprüche

1. Verfahren zur Herstellung von monomeren oder dimeren Proteinen oder Peptiden, enthaltend interne oder externe Disulfidbrücken mit den folgenden Verfahrensstufen:
a) es wird eine zellfreies Lysat, erhältlich aus eukaryotischen Zellen, bereit gestellt, welches funktionale mikrosomale Vesikel enthält,
b) zum Lysat wird eine Nukleinsäure zugegeben, welche das Protein oder Peptid codiert und zusätzlich eine Signalsequenz enthält,
c) das Lysat mit der Nukleinsäure wird für eine vorgegebene Zeit auf einer Temperatur im Bereich von 20-35°C gehalten, wobei mit der Nukleinsäure gebildete Proteine oder Peptide in die mikrosomalen Vesikel translociert werden,
d) die mikrosomalen Vesikel werden anschließend aufgelöst und die dabei erhaltenen Proteine oder Peptide werden optional von dem Lysat abgetrennt.

2. Verfahren nach Anspruch 1, wobei das Lysat keine Chaperone und/oder Proteindisulfidisomerasen enthält, oder wobei dem Lysat keine Chaperone und/oder Proteindisulfidisomerasen zugesetzt werden.

3. Verfahren nach Anspruch 1 oder 2, wobei die Proteine oder Peptide vorzugsweise dimere monoklonalen Antikörper oder physiologisch wirksame vorzugsweise dimere Antikörperfragmente sind.

4. Verfahren nach einem der Ansprüche 1 bis 3, wobei das Lysat keiner chemischen Vorbehandlung zur Stabilisierung des Redox-Potentials unterzogen ist.

5. Verfahren nach einem der Ansprüche 1 bis 4, wobei die eukaryotischen Zellen Insektenzellen sind.

6. Verfahren nach einem der Ansprüche 1 bis 5, wobei die Stufe c) unterhalb einer Temperatur von 30 °C, insbesondere unterhalb von 26 °C, durchgeführt wird.

7. Verfahren nach einem der Ansprüche 1 bis 6, wobei die Stufe c) für eine Dauer von 0,5 bis 5 h durchgeführt wird.

8. Verfahren nach einem der Ansprüche 1 bis 7, wobei die Nukleinsäure eine lineare DNA ist.

9. Proteine oder Peptide oder Zubereitung enthaltend Proteine oder Peptide, erhältlich mit einem Verfahren nach einem der Ansprüche 1 bis 8.

10. Zubereitung enthaltend Proteine oder Peptide und ein zellfreies Lysat, erhältlich aus eukaryotischen Zellen, wobei das Lysat funktionale mikrosomale Vesikel oder Produkte einer Auflösung solcher mikrosomalen Vesikel enthält, und wobei die Zubereitung keine endogenen und/oder zugesetzten Chaperone und/oder Proteindisulfidisomerasen enthält.


SEQUENCE LISTING

<110> RiNA Netzwerk RNA Technologien GmbH
MERK, Dr. Helmut
GERRITS, Dr. Michael
GLESS, Christine
STIEGE, Dr. Wolfgang

<120> Verfahren zur Herstellung von Proteinen und Peptiden

<130> RNA-EP-1201

<150> DE 10 2011 107 562.7
<151> 2011-07-11

<160> 6

<170> PatentIn version 3.3

<210> 1
<211> 3774
<212> DNA
<213> Artificial

<220>
<223> Plasmid

<400> 1
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gcttgcatgc aaggagatgg 420

cgcccaacag tcccccggcc acggggcctg ccaccatacc cacgccgaaa caagcgctca 480

tgagcccgaa gtggcgagcc cgatcttccc catcggtgat gtcggcgata taggcgccag 540

caaccgcacc tgtggcgccg gtgatgccgg ccacgatgcg tccggcgtag aggatcgaga 600

tcgatctcga tcccgcgaaa ttaatacgac tcactatagg gagaccacaa cggtttccct 660

ctagaaataa ttttgtttaa ctttaagaag gagatatacc atgaaattct tagtcaacgt 720

tgccctggtt tttatggtgg tgtatattag ctatatttat gccgatattg tgctgaccca 780

gagtccggca accctgagcg ttacaccggg taatagcgtt agcctgagct gtcgtgcaag 840

ccagagcatt ggtaataatc tgcattggta tcagcagaaa agccatgaaa gtccgcgtct 900

gctgattaaa tatgcaagcc agtcaattag cggtattccg agccgtttta gcggtagcgg 960

tagtggcacc gattttaccc tgagcattaa tagcgttgaa accgaagatt ttggcatgta 1020

tttttgccag cagagcaata gctggccgta tacctttggt ggtggcacca aactggaaat 1080

taaacgtgca gatgcagcac cgaccgttag cattttcccg ccgagcagcg aacagctgac 1140

cagcggtggt gcaagcgttg tttgttttct gaataacttt tatccgaaag atatcaatgt 1200

gaaatggaaa attgatggca gcgaacgtca gaatggtgtt ctgaatagct ggaccgatca 1260
ggatagcaaa gatagcacct atagcatgag cagcaccctg accctgacca aagatgaata 1320
tgaacgccat aatagctata cctgtgaagc aacccataaa accagcacca gcccgattgt 1380
taaaagcttt aatcgcaatg aatgctaata actcgagcgg ccgcctgcag atctaaataa 1440
taagtaatta actagtgagc aataactagc ataacccctt ggggcctcta aacgggtctt 1500
gaggggtttt ttgatatccc gggatttaaa taggcctgaa ttcgtaatca tggtcatagc 1560
tgtttcctgt gtgaaattgt tatccgctca caattccaca caacatacga gccggaagca 1620
taaagtgtaa agcctggggt gcctaatgag tgagctaact cacattaatt gcgttgcgct 1680
cactgcccgc tttccagtcg ggaaacctgt cgtgccagct gcattaatga atcggccaac 1740
gcgcggggag aggcggtttg cgtattgggc gctcttccgc ttcctcgctc actgactcgc 1800
tgcgctcggt cgttcggctg cggcgagcgg tatcagctca ctcaaaggcg gtaatacggt 1860
tatccacaga atcaggggat aacgcaggaa agaacatgtg agcaaaaggc cagcaaaagg 1920
ccaggaaccg taaaaaggcc gcgttgctgg cgtttttcca taggctccgc ccccctgacg 1980
agcatcacaa aaatcgacgc tcaagtcaga ggtggcgaaa cccgacagga ctataaagat 2040
accaggcgtt tccccctgga agctccctcg tgcgctctcc tgttccgacc ctgccgctta 2100
ccggatacct gtccgccttt ctcccttcgg gaagcgtggc gctttctcat agctcacgct 2160
gtaggtatct cagttcggtg taggtcgttc gctccaagct gggctgtgtg cacgaacccc 2220
ccgttcagcc cgaccgctgc gccttatccg gtaactatcg tcttgagtcc aacccggtaa 2280
gacacgactt atcgccactg gcagcagcca ctggtaacag gattagcaga gcgaggtatg 2340
taggcggtgc tacagagttc ttgaagtggt ggcctaacta cggctacact agaaggacag 2400
tatttggtat ctgcgctctg ctgaagccag ttaccttcgg aaaaagagtt ggtagctctt 2460
gatccggcaa acaaaccacc gctggtagcg gtggtttttt tgtttgcaag cagcagatta 2520
cgcgcagaaa aaaaggatct caagaagatc ctttgatctt ttctacgggg tctgacgctc 2580
agtggaacga aaactcacgt taagggattt tggtcatgag attatcaaaa aggatcttca 2640
cctagatcct tttaaattaa aaatgaagtt ttaaatcaat ctaaagtata tatgagtaaa 2700
cttggtctga cagttaccaa tgcttaatca gtgaggcacc tatctcagcg atctgtctat 2760
ttcgttcatc catagttgcc tgactccccg tcgtgtagat aactacgata cgggagggct 2820
taccatctgg ccccagtgct gcaatgatac cgcgagatcc acgctcaccg gctccagatt 2880
tatcagcaat aaaccagcca gccggaaggg ccgagcgcag aagtggtcct gcaactttat 2940
ccgcctccat ccagtctatt aattgttgcc gggaagctag agtaagtagt tcgccagtta 3000
atagtttgcg caacgttgtt gccattgcta caggcatcgt ggtgtcacgc tcgtcgtttg 3060
gtatggcttc attcagctcc ggttcccaac gatcaaggcg agttacatga tcccccatgt 3120
tgtgcaaaaa agcggttagc tccttcggtc ctccgatcgt tgtcagaagt aagttggccg 3180
cagtgttatc actcatggtt atggcagcac tgcataattc tcttactgtc atgccatccg 3240

taagatgctt ttctgtgact ggtgagtact caaccaagtc attctgagaa tagtgtatgc 3300

ggcgaccgag ttgctcttgc ccggcgtcaa tacgggataa taccgcgcca catagcagaa 3360

ctttaaaagt gctcatcatt ggaaaacgtt cttcggggcg aaaactctca aggatcttac 3420

cgctgttgag atccagttcg atgtaaccca ctcgtgcacc caactgatct tcagcatctt 3480

ttactttcac cagcgtttct gggtgagcaa aaacaggaag gcaaaatgcc gcaaaaaagg 3540

gaataagggc gacacggaaa tgttgaatac tcatactctt cctttttcaa tattattgaa 3600

gcatttatca gggttattgt ctcatgagcg gatacatatt tgaatgtatt tagaaaaata 3660

aacaaatagg ggttccgcgc acatttcccc gaaaagtgcc acctgacgtc taagaaacca 3720

ttattatcat gacattaacc tataaaaata ggcgtatcac gaggcccttt cgtc 3774

<210> 2
<211> 3777
<212> DNA
<213> Artificial

<220>
<223> Plasmid

<400> 2
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gcttgcatgc aaggagatgg 420

cgcccaacag tcccccggcc acggggcctg ccaccatacc cacgccgaaa caagcgctca 480

tgagcccgaa gtggcgagcc cgatcttccc catcggtgat gtcggcgata taggcgccag 540

caaccgcacc tgtggcgccg gtgatgccgg ccacgatgcg tccggcgtag aggatcgaga 600

tcgatctcga tcccgcgaaa ttaatacgac tcactatagg gagaccacaa cggtttccct 660

ctagaaataa ttttgtttaa ctttaagaag gagatatacc atgaaattct tagtcaacgt 720

tgccctggtt tttatggtgg tgtatattag ctatatttat gccgatgtgc agctgcagga 780

aagcggtccg agcctggtta aaccgagcca gaccctgagc ctgacctgta gcgttaccgg 840

tgatagcatt accagcgatt attggagctg gattcgtaaa tttccgggta atcgtctgga 900

atatatgggt tatgttagct atagcggcag cacctattat aatccgagcc tgaaaagccg 960

tattagcatt acccgtgata ccagcaaaaa tcagtattat ctggatctga atagcgtgac 1020

caccgaagat accgcaacct attactgtgc aaattgggat ggtgattatt ggggtcaggg 1080

caccctggtt accgttagcg cagcaaaaac cacaccgccg agcgtttatc cgctggcacc 1140

gggtagcgca gcacagacca atagcatggt taccctgggt tgtctggtga aaggttattt 1200

tccggaaccg gttaccgtta cctggaatag cggtagcctg agcagcggtg ttcatacctt 1260
tccggcagtt ctgcagagcg atctgtatac cctgagcagc agcgttaccg ttccgagcag 1320
tccgcgtccg agcgaaaccg ttacctgtaa tgttgcacat ccggcaagca gcaccaaagt 1380
tgataaaaaa attgttccgc gtgattgcta ataactcgag cggccgcctg cagatctaaa 1440
taataagtaa ttaactagtg agcaataact agcataaccc cttggggcct ctaaacgggt 1500
cttgaggggt tttttgatat cccgggattt aaataggcct gaattcgtaa tcatggtcat 1560
agctgtttcc tgtgtgaaat tgttatccgc tcacaattcc acacaacata cgagccggaa 1620
gcataaagtg taaagcctgg ggtgcctaat gagtgagcta actcacatta attgcgttgc 1680
gctcactgcc cgctttccag tcgggaaacc tgtcgtgcca gctgcattaa tgaatcggcc 1740
aacgcgcggg gagaggcggt ttgcgtattg ggcgctcttc cgcttcctcg ctcactgact 1800
cgctgcgctc ggtcgttcgg ctgcggcgag cggtatcagc tcactcaaag gcggtaatac 1860
ggttatccac agaatcaggg gataacgcag gaaagaacat gtgagcaaaa ggccagcaaa 1920
aggccaggaa ccgtaaaaag gccgcgttgc tggcgttttt ccataggctc cgcccccctg 1980
acgagcatca caaaaatcga cgctcaagtc agaggtggcg aaacccgaca ggactataaa 2040
gataccaggc gtttccccct ggaagctccc tcgtgcgctc tcctgttccg accctgccgc 2100
ttaccggata cctgtccgcc tttctccctt cgggaagcgt ggcgctttct catagctcac 2160
gctgtaggta tctcagttcg gtgtaggtcg ttcgctccaa gctgggctgt gtgcacgaac 2220
cccccgttca gcccgaccgc tgcgccttat ccggtaacta tcgtcttgag tccaacccgg 2280
taagacacga cttatcgcca ctggcagcag ccactggtaa caggattagc agagcgaggt 2340
atgtaggcgg tgctacagag ttcttgaagt ggtggcctaa ctacggctac actagaagga 2400
cagtatttgg tatctgcgct ctgctgaagc cagttacctt cggaaaaaga gttggtagct 2460
cttgatccgg caaacaaacc accgctggta gcggtggttt ttttgtttgc aagcagcaga 2520
ttacgcgcag aaaaaaagga tctcaagaag atcctttgat cttttctacg gggtctgacg 2580
ctcagtggaa cgaaaactca cgttaaggga ttttggtcat gagattatca aaaaggatct 2640
tcacctagat ccttttaaat taaaaatgaa gttttaaatc aatctaaagt atatatgagt 2700
aaacttggtc tgacagttac caatgcttaa tcagtgaggc acctatctca gcgatctgtc 2760
tatttcgttc atccatagtt gcctgactcc ccgtcgtgta gataactacg atacgggagg 2820
gcttaccatc tggccccagt gctgcaatga taccgcgaga cccacgctca ccggctccag 2880
atttatcagc aataaaccag ccagccggaa gggccgagcg cagaagtggt cctgcaactt 2940
tatccgcctc catccagtct attaattgtt gccgggaagc tagagtaagt agttcgccag 3000
ttaatagttt gcgcaacgtt gttgccattg ctacaggcat cgtggtgtca cgctcgtcgt 3060
ttggtatggc ttcattcagc tccggttccc aacgatcaag gcgagttaca tgatccccca 3120
tgttgtgcaa aaaagcggtt agctccttcg gtcctccgat cgttgtcaga agtaagttgg 3180
ccgcagtgtt atcactcatg gttatggcag cactgcataa ttctcttact gtcatgccat 3240

ccgtaagatg cttttctgtg actggtgagt actcaaccaa gtcattctga gaatagtgta 3300

tgcggcgacc gagttgctct tgcccggcgt caatacggga taataccgcg ccacatagca 3360

gaactttaaa agtgctcatc attggaaaac gttcttcggg gcgaaaactc tcaaggatct 3420

taccgctgtt gagatccagt tcgatgtaac ccactcgtgc acccaactga tcttcagcat 3480

cttttacttt caccagcgtt tctgggtgag caaaaacagg aaggcaaaat gccgcaaaaa 3540

agggaataag ggcgacacgg aaatgttgaa tactcatact cttccttttt caatattatt 3600

gaagcattta tcagggttat tgtctcatga gcggatacat atttgaatgt atttagaaaa 3660

ataaacaaat aggggttccg cgcacatttc cccgaaaagt gccacctgac gtctaagaaa 3720

ccattattat catgacatta acctataaaa ataggcgtat cacgaggccc tttcgtc 3777

<210> 3
<211> 3465
<212> DNA
<213> Artificial

<220>
<223> Plasmid

<400> 3
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gctttctcga gcggccgcta 420

gctaatacga ctcactatag ggagaccaca acggtttccc tctagaaata attttgttta 480

actttaagaa ggagataaac aatggatatt gtgctgaccc agagtccggc aaccctgagc 540

gttacaccgg gtaatagcgt tagcctgagc tgtcgtgcaa gccagagcat tggtaataat 600

ctgcattggt atcagcagaa aagccatgaa agtccgcgtc tgctgattaa atatgcaagc 660

cagtcaatta gcggtattcc gagccgtttt agcggtagcg gtagtggcac cgattttacc 720

ctgagcatta atagcgttga aaccgaagat tttggcatgt atttttgcca gcagagcaat 780

agctggccgt atacctttgg tggtggcacc aaactggaaa ttaaacgtgc agatgcagca 840

ccgaccgtta gcatttttcc gccgagcagc gaacagctga ccagcggtgg tgcaagcgtt 900

gtttgttttc tgaataactt ttatccgaaa gatatcaatg tgaaatggaa aattgatggc 960

agcgaacgtc agaatggtgt tctgaatagc tggaccgatc aggatagcaa agatagcacc 1020

tatagcatga gcagcaccct gaccctgacc aaagatgaat atgaacgcca taatagctat 1080

acctgtgaag caacccataa aaccagcacc agcccgattg ttaaaagctt taatcgcaat 1140

gaatgctaat aactaactaa ccaagatctg taccccttgg ggcctctaaa cgggtcttga 1200

gggggttttt ggatccgaat tcaccggtgc aattcgtaat catgtcatag ctgtttcctg 1260
tgtgaaattg ttatccgctc acaattccac acaacatacg agccggaagc ataaagtgta 1320
aagcctgggg tgcctaatga gtgagctaac tcacattaat tgcgttgcgc tcactgcccg 1380
ctttccagtc gggaaacctg tcgtgccagc tgcattaatg aatcggccaa cgcgcgggga 1440
gaggcggttt gcgtattggg cgctcttccg cttcctcgct cactgactcg ctgcgctcgg 1500
tcgttcggct gcggcgagcg gtatcagctc actcaaaggc ggtaatacgg ttatccacag 1560
aatcagggga taacgcagga aagaacatgt gagcaaaagg ccagcaaaag gccaggaacc 1620
gtaaaaaggc cgcgttgctg gcgtttttcc ataggctccg cccccctgac gagcatcaca 1680
aaaatcgacg ctcaagtcag aggtggcgaa acccgacagg actataaaga taccaggcgt 1740
ttccccctgg aagctccctc gtgcgctctc ctgttccgac cctgccgctt accggatacc 1800
tgtccgcctt tctcccttcg ggaagcgtgg cgctttctca tagctcacgc tgtaggtatc 1860
tcagttcggt gtaggtcgtt cgctccaagc tgggctgtgt gcacgaaccc cccgttcagc 1920
ccgaccgctg cgccttatcc ggtaactatc gtcttgagtc caacccggta agacacgact 1980
tatcgccact ggcagcagcc actggtaaca ggattagcag agcgaggtat gtaggcggtg 2040
ctacagagtt cttgaagtgg tggcctaact acggctacac tagaagaaca gtatttggta 2100
tctgcgctct gctgaagcca gttaccttcg gaaaaagagt tggtagctct tgatccggca 2160
aacaaaccac cgctggtagc ggtggttttt ttgtttgcaa gcagcagatt acgcgcagaa 2220
aaaaaggatc tcaagaagat cctttgatct tttctacggg gtctgacgct cagtggaacg 2280
aaaactcacg ttaagggatt ttggtcatga gattatcaaa aaggatcttc acctagatcc 2340
ttttaaatta aaaatgaagt tttaaatcaa tctaaagtat atatgagtaa acttggtctg 2400
acagttacca atgcttaatc agtgaggcac ctatctcagc gatctgtcta tttcgttcat 2460
ccatagttgc ctgactcccc gtcgtgtaga taactacgat acgggagggc ttaccatctg 2520
gccccagtgc tgcaatgata ccgcgagacc cacgctcacc ggctccagat ttatcagcaa 2580
taaaccagcc agccggaagg gccgagcgca gaagtggtcc tgcaacttta tccgcctcca 2640
tccagtctat taattgttgc cgggaagcta gagtaagtag ttcgccagtt aatagtttgc 2700
gcaacgttgt tgccattgct acaggcatcg tggtgtcacg ctcgtcgttt ggtatggctt 2760
cattcagctc cggttcccaa cgatcaaggc gagttacatg atcccccatg ttgtgcaaaa 2820
aagcggttag ctccttcggt cctccgatcg ttgtcagaag taagttggcc gcagtgttat 2880
cactcatggt tatggcagca ctgcataatt ctcttactgt catgccatcc gtaagatgct 2940
tttctgtgac tggtgagtac tcaaccaagt cattctgaga atagtgtatg cggcgaccga 3000
gttgctcttg cccggcgtca atacgggata ataccgcgcc acatagcaga actttaaaag 3060
tgctcatcat tggaaaacgt tcttcggggc gaaaactctc aaggatctta ccgctgttga 3120
gatccagttc gatgtaaccc actcgtgcac ccaactgatc ttcagcatct tttactttca 3180
ccagcgtttc tgggtgagca aaaacaggaa ggcaaaatgc cgcaaaaaag ggaataaggg 3240

cgacacggaa atgttgaata ctcatactct tcctttttca atattattga agcatttatc 3300

agggttattg tctcatgagc ggatacatat ttgaatgtat ttagaaaaat aaacaaatag 3360

gggttccgcg cacatttccc cgaaaagtgc cacctgacgt ctaagaaacc attattatca 3420

tgacattaac ctataaaaat aggcgtatca cgaggccctt tcgtc 3465

<210> 4
<211> 3518
<212> DNA
<213> Artificial

<220>
<223> Plasmid

<400> 4
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360

tttcccagtc acgacgttgt aaaacgacgg ccagtgccaa gctttctcga gcggccgcta 420

gctaatacga ctcactatag ggagaccaca acggtttccc tctagaaata attttgttta 480

actttaagaa ggagataaac aatggatgtg cagctgcagg aaagcggtcc gagcctggtt 540

aaaccgagcc agaccctgag cctgacctgt agcgttaccg gtgatagcat taccagcgat 600

tattggagct ggattcgtaa atttccgggt aatcgtctgg aatatatggg ttatgttagc 660

tatagcggca gcacctatta taatccgagc ctgaaaagcc gtattagcat tacccgtgat 720

accagcaaaa atcagtatta tctggatctg aatagcgtga ccaccgaaga taccgcaacc 780

tattactgtg caaattggga tggtgattat tggggtcagg gcaccctggt taccgttagc 840

gcagcaaaaa ccacaccgcc gagcgtttat ccgctggcac cgggtagcgc agcacagacc 900

aatagcatgg ttaccctggg ttgtctggtg aaaggttatt ttccggaacc ggttaccgtt 960

acctggaata gcggtagcct gagcagcggt gttcatacct ttccggcagt tctgcagagc 1020

gatctgtata ccctgagcag cagcgttacc gttccgagca gtccgcgtcc gagcgaaacc 1080

gttacctgta atgttgcaca tccggcaagc agcaccaaag ttgataaaaa aattgttccg 1140

cgtgattgct aataactcga gcggccgcct gcagatctaa ataataagta attaactagt 1200

gagcaataac tagcataacc ccttggggcc tctaaacggg tcttgagggg ttttttgata 1260

tcccgggatt taaataggcc tgaattcgta atcatggtca tagctgtttc ctgtgtgaaa 1320

ttgttatccg ctcacaattc cacacaacat acgagccgga agcataaagt gtaaagcctg 1380

gggtgcctaa tgagtgagct aactcacatt aattgcgttg cgctcactgc ccgctttcca 1440

gtcgggaaac ctgtcgtgcc agctgcatta atgaatcggc caacgcgcgg ggagaggcgg 1500

tttgcgtatt gggcgctctt ccgcttcctc gctcactgac tcgctgcgct cggtcgttcg 1560
gctgcggcga gcggtatcag ctcactcaaa ggcggtaata cggttatcca cagaatcagg 1620
ggataacgca ggaaagaaca tgtgagcaaa aggccagcaa aaggccagga accgtaaaaa 1680
ggccgcgttg ctggcgtttt tccataggct ccgcccccct gacgagcatc acaaaaatcg 1740
acgctcaagt cagaggtggc gaaacccgac aggactataa agataccagg cgtttccccc 1800
tggaagctcc ctcgtgcgct ctcctgttcc gaccctgccg cttaccggat acctgtccgc 1860
ctttctccct tcgggaagcg tggcgctttc tcatagctca cgctgtaggt atctcagttc 1920
ggtgtaggtc gttcgctcca agctgggctg tgtgcacgaa ccccccgttc agcccgaccg 1980
ctgcgcctta tccggtaact atcgtcttga gtccaacccg gtaagacacg acttatcgcc 2040
actggcagca gccactggta acaggattag cagagcgagg tatgtaggcg gtgctacaga 2100
gttcttgaag tggtggccta actacggcta cactagaagg acagtatttg gtatctgcgc 2160
tctgctgaag ccagttacct tcggaaaaag agttggtagc tcttgatccg gcaaacaaac 2220
caccgctggt agcggtggtt tttttgtttg caagcagcag attacgcgca gaaaaaaagg 2280
atctcaagaa gatcctttga tcttttctac ggggtctgac gctcagtgga acgaaaactc 2340
acgttaaggg attttggtca tgagattatc aaaaaggatc ttcacctaga tccttttaaa 2400
ttaaaaatga agttttaaat caatctaaag tatatatgag taaacttggt ctgacagtta 2460
ccaatgctta atcagtgagg cacctatctc agcgatctgt ctatttcgtt catccatagt 2520
tgcctgactc cccgtcgtgt agataactac gatacgggag ggcttaccat ctggccccag 2580
tgctgcaatg ataccgcgag atccacgctc accggctcca gatttatcag caataaacca 2640
gccagccgga agggccgagc gcagaagtgg tcctgcaact ttatccgcct ccatccagtc 2700
tattaattgt tgccgggaag ctagagtaag tagttcgcca gttaatagtt tgcgcaacgt 2760
tgttgccatt gctacaggca tcgtggtgtc acgctcgtcg tttggtatgg cttcattcag 2820
ctccggttcc caacgatcaa ggcgagttac atgatccccc atgttgtgca aaaaagcggt 2880
tagctccttc ggtcctccga tcgttgtcag aagtaagttg gccgcagtgt tatcactcat 2940
ggttatggca gcactgcata attctcttac tgtcatgcca tccgtaagat gcttttctgt 3000
gactggtgag tactcaacca agtcattctg agaatagtgt atgcggcgac cgagttgctc 3060
ttgcccggcg tcaatacggg ataataccgc gccacatagc agaactttaa aagtgctcat 3120
cattggaaaa cgttcttcgg ggcgaaaact ctcaaggatc ttaccgctgt tgagatccag 3180
ttcgatgtaa cccactcgtg cacccaactg atcttcagca tcttttactt tcaccagcgt 3240
ttctgggtga gcaaaaacag gaaggcaaaa tgccgcaaaa aagggaataa gggcgacacg 3300
gaaatgttga atactcatac tcttcctttt tcaatattat tgaagcattt atcagggtta 3360
ttgtctcatg agcggataca tatttgaatg tatttagaaa aataaacaaa taggggttcc 3420
gcgcacattt ccccgaaaag tgccacctga cgtctaagaa accattatta tcatgacatt 3480
aacctataaa aataggcgta tcacgaggcc ctttcgtc 3518

<210> 5
<211> 931
<212> DNA
<213> Artificial

<220>
<223> Plasmid

<400> 5
atgatatctc gagcggccgc tagctaatac gactcactat agggagacca caacggtttc 60

cctctagaaa taattttgtt taactttaag aaggagataa acaatgaaat tcttagtcaa 120

cgttgccctt gtttttatgg tcgtatacat ttcttacatc tatgcggacg atatccagat 180

gacccagtct ccggcttctc tgtctgcttc tgttggtgaa accgttacct tcacctgccg 240

tgcttctgaa atgatctact cttacctggc ttggtatcag cagaaacagg gtaaatctcc 300

gcaactgctg gttcacgacg ctaaaaccct ggctgaaggt gttccgtccc gtttctctgg 360

tggtggttct ggcacccagt tctctctgaa aatcaacacc ctccagccgg aagacttcgg 420

tacctactac tgccagcacc actacggtaa cccgccgacc ttcggtggtg gcaccaaact 480

cgagatcaaa cgggggatcg ctgcaccatc tgtcttcatc ttcccgccat ctgatgagca 540

gttgaaatct ggaactgcct ctgttgtgtg cctgctgaat aacttctatc ccagagaggc 600

caaagtacag tggaaggtgg ataacgccct ccaatcgggt aactcccagg agagtgtcac 660

agagcaggac agcaaggaca gcacctacag cctcagcagc accctgacgc tgagcaaagc 720

agactacgag aaacacaaag tctacgcctg cgaagtcacc catcagggcc tgagttcgcc 780

cgtcacaaag agcttcaacc gcggagagtg ttctgcctgg tctcatccgc aattcgaaaa 840

ataataacta actaaccaag atctgtaccc cttggggcct ctaaacgggt cttgaggggt 900

tttttggatc cgaattcacc ggtgatatca t 931

<210> 6
<211> 919
<212> DNA
<213> Artificial

<220>
<223> Plasmid

<400> 6
atgatatctc gagcggccgc tagctaatac gactcactat agggagacca caacggtttc 60

cctctagaaa taattttgtt taactttaag aaggagataa acaatgaaat tcttagtcaa 120

cgttgccctt gtttttatgg tcgtatacat ttcttacatc tatgcggacc aggttcagct 180

gaaacagtct ggtccgggtc ttgtacagcc gtcccagtct ctgtctatca cctgcaccgt 240

ttccggattc tctctgacca ccttcggtgt tcactgggtt cgtcagtccc cgggtaaagg 300

tctggaatgg ctgggtgtta tctggcgttc tggtatcacc gactacaacg ttccgttcat 360

gtctcgtctg tctatcacca aagacaactc taaatctcag gttttcttca aactgaactc 420

tctgcaaccg gacgacaccg ctatctacta ctgcgctaaa aacgatccgg gtaccggttt 480

cgcttactgg ggtcagggca ccctggtcac cgtttctgca gggagcacca agggcccatc 540

ggtcttcccc ctggcaccct cctccaagag cacctctggg ggcacagcgg ccctgggctg 600

cctggtcaag gactacttcc ccgaaccggt gacggtgtcg tggaactcag gcgccctgac 660

cagcggcgtg cacaccttcc cggctgtcct acagtcctca ggactctact ccctcagcag 720

cgtggtgact gtgccctcca gcagcttggg cacccagacc tacatctgca acgttaatca 780

caaacccagc aacaccaagg tcgacaagaa agttgagccc aaatcttgct aataactaac 840

taaccaagat ctgtacccct tggggcctct aaacgggtct tgaggggttt tttggatccg 900

aattcaccgg tgatatcat 919